# EUROPEAN PATENT SPECIFICATION

(11) **EP 3 516 394 B1**
(45) Date of publication and mention of the grant of the patent: **21.06.2023**
(21) Application number: 17859333.1
(22) Date of filing: 22.11.2017
(51) Int. Cl.: G01N 33/543, G01N 33/569, G01N 33/53, C07K 14/35, G01N 33/68, G01N 33/50

(54) **MYCOBACTERIUM TUBERCULOSIS PROTEINS IN DIAGNOSTIC ASSAYS AND DEVICES FOR TUBERCULOSIS DETECTION AND DIAGNOSIS**
MYCOBACTERIUM-TUBERCULOSIS-PROTEINE IN DIAGNOSTISCHEN TESTS UND VORRICHTUNGEN ZUR ERKENNUNG UND DIAGNOSE VON TUBERKULOSE
PROTÉINES DE MYCOBACTERIUM TUBERCULOSIS DANS DES DOSAGES ET DISPOSITIFS DE DIAGNOSTIC POUR LA DÉTECTION ET LE DIAGNOSTIC DE LA TUBERCULOSE

(43) Date of publication of application: 31.07.2019
(73) Proprietor: Pace Diagnostics, Inc, Monrovia, CA 91016 (US)
(72) Inventor: NGUYEN, Hiep-Hoa, T., Santa Ana, CA 92707 (US)
(74) Representative: Pföstl, Andreas
(86) International application number: PCT/US2017/063154
(87) International publication number: WO 2018/068064

(56) References cited:
- WO-A2-02/074903
- US-A1- 2003 165 525
- US-A1- 2007 141 087
- US-A1- 2008 171 345
- US-A1- 2008 267 990
- US-A1- 2015 219 645
- LIYA WASSIE ET AL: "Development of a proof of concept immunochromatographic lateral flow assay for point of care diagnosis of Mycobacterium tuberculosis", BMC RESEARCH NOTES, BIOMED CENTRAL LTD, GB, vol. 6, no. 1, 21 May 2013 (2013-05-21), page 202, XP021152681, ISSN: 1756-0500, DOI: 10.1186/1756-0500-6-202
- Karin Weldingh, Ida Rosenkrands, Limei Meng Okkels, T. Mark Doherty, and Peter Andersen: "Assessing the Serodiagnostic Potential of 35 Mycobacterium tuberculosisProteins and Identification of Four Novel Serological Antigens", , 1 January 2005 (2005-01-01), XP055006745, Retrieved from the Internet: URL:http://jcm.asm.org/cgi/reprint/43/1/57 .pdf [retrieved on 2011-09-09]

## Description

### CROSS-REFERENCE TO RELATED APPLICATIONS

This application claims the benefit of U.S. Provisional Appl. No. 62/398,213, filed September 22, 2016.

### FIELD OF THE INVENTION

The invention relates to infectious disease, in particular to medical diagnostics and methods of detecting tuberculosis infections.

### BACKGROUND OF THE INVENTION

Traditionally, the diagnosis of tuberculosis (TB) is based on signs and symptoms suggestive of TB together with appropriate chest x-ray changes and demonstration of mycobacteria in the sputum (smear-positive TB) and positive cultures for TB. In order to get a positive smear, the number of TB organisms in the sputum sample must exceed certain detection threshold. TB cultures are often slow and laborious, and many labs may not have the appropriate facilities and expertise. As a result, a TB skin test, the tuberculin skin test (TST) is often used in addition to smears and cultures to establish a TB diagnosis in patients with relevant symptoms and x-ray changes. The tuberculin skin test could be used to diagnose latent tuberculosis, but it requires two visits and skilled personnel for test placement and interpretation. In addition, the tuberculin skin test often does not separate latent tuberculosis infection from prior immunization with *Mycobacterium bovis* Bacille Calmette Guerin (BCG) or infection with environmental mycobacteria since the TST utilizes tuberculin purified protein derivatives (PPD) as the antigen components. A major advance was the identification of early secreted antigenic target 6 kD protein (ESAT-6) and culture filtrate protein 10 (CFP10). ESAT-6 and CFP-10 are secreted by all *M. tuberculosis* and pathogenic *M. bovis* strains but are absent from all Bacille Calmette-Guérin (BCG) vaccine strains and from commonly encountered nontuberculous mycobacteria (NTM) except *M. kansasii, M. szulgai, and M. marinum.* Colangeli et al., Infect. Immun. 2000; 68:990-993; Harboe et al., Infect. Immun. 1996; 64:16-22.

Given the cumbersome nature of these assays - smears, cultures and TST, - time consuming, requiring unique expertise and facility (culturing), return visit to the clinics (TST), there have been intense interests at other blood markers that could be used to establish the diagnosis of TB. Advances in these areas have led to at least two commercially available assays - TSPOT-TB and QuantiFeronTB-Gold. Both of these tests essentially detect blood T-cells that react with antigens (ESAT6 and CFP10) specific to *M. tuberculosis.* These tests involve stimulating blood lymphocytes with the antigens in the form of peptides corresponding to the ESAT6 and CFP10 or the full length recombinant antigens, followed by measurement of interferon-γ (IFN-γ) secreted by the T-cells by enzyme-linked immunoassay (QuantiFeronTB-Gold) or by detection of interferon-γ-producing cells by enzyme-linked immunospot assay (TSPOT-TB).

While these T-cell based TB tests in ELISA format are a definite improvement over the TST in the diagnosis of TB, there are still a number of caveats and issues. For instance, the laboratory intensive nature of these IGRAs which are based on the ELISA format is problematic, especially under resource poor settings. An alternative to T-cell based tests are assays based on the detection of tuberculosis-specific antibodies.

A serological test with appropriate sensitivity and specificity to detect tuberculosis-specific antibodies in blood/serum/saliva samples can have significant advantages over currently available tests. Unlike the tuberculin skin test (TST), assays of antibody responses to *M. tuberculosis* antigens is less invasive and dangerous (adverse effects) and saves time. These tests also do not require the patient to return to the clinic for evaluation. These advantages are similar to those of IGRAs. In addition, the serological assay to detect TB-specific antibodies can also be casted into user-friendly and field-ready rapid formats. These rapid formats can reduce the heavy laboratory dependence of T-cell based assays. The responses with rapid formats can also be calibrated in such a way that the ambiguity in result interpretation might be avoided as found in previous studies. Liebeschuetz et al. Lancet 364(9452):2196-203; Goodwin, D.J. (2010) Operational issues for QFT-GIT testing. Tri-Service TB screening and testing policy review. Joint Preventive Medicine Policy Group. Rosslyn, VA; Oct 2010. (88ABW-2010-5700). This process can be achieved in rapid tests by tuning, i.e. positive responses observed only above certain predetermined threshold. The observable threshold is determined through extensive screening with field samples.

One major issue with the serological assay to detect TB-specific antibodies is which antigen(s) can be used in the assay. Over the past hundred years, our understanding of the antibody response has changed dramatically. Also, new antigens have been identified and several recombinant antigens have been produced. However, the accuracy of existing serological assays is modest, and these tests have not been clinically useful. Steingart et al. Thorax 2007; 62:911-8; Steingart et al., PLoS Med. 2007; 4:e202; Steingart et al., Clin. Vaccine Immunol. 2009; 16:260-76; Abebe et al., Scand. J. Immunol. 2007; 66:176-91. Nevertheless, efforts towards discovery of novel additional antigens and better methodological strategies for developing efficient serological assays have continued.

There is a significant need to develop new assays and screens to detect tuberculosis infections in patients.

This background information is provided for informational purposes only. No admission is necessarily intended, nor should it be construed, that any of the preceding information constitutes prior art against the present invention.

### SUMMARY

In one aspect, the invention relates to a protein combination that can be used in diagnostics to detect tuberculosis.

The disclosure provides isolated Rv0222 protein or protein(s) with high sequence identity (>80% sequence identity), whole or immunogenic peptides to be used singly or in combination with other antigens in diagnostics/detection devices to detect antibodies which specifically bind to Rv0222 and which are present in a bodily fluid sample (serum, whole blood, plasma, saliva).

The disclosure provides isolated Rv3120 protein or protein(s) with high sequence identity (>80% sequence identity), to be used singly or in combination with other antigens in diagnostics/detection devices to detect antibodies which specifically bind to Rv3120 and which are present in a bodily fluid sample (serum, whole blood, plasma, saliva).

The disclosure provides isolated Rv1989c protein or protein(s) with high sequence identity (>80% sequence identity), to be used singly or in combination with other antigens in diagnostics/detection devices to detect antibodies which specifically bind to Rv1989c and which are present in a bodily fluid sample (serum, whole blood, plasma, saliva).

The disclosure provides isolated Rv3118 protein or protein(s) with high sequence identity (>80% sequence identity), whole or immunogenic peptides to be used singly or in combination with other antigens in diagnostics/detection devices to detect antibodies which specifically bind to Rv3118 and which are present in a bodily fluid sample (serum, whole blood, plasma, saliva).

The disclosure provides isolated Rv1636 protein or protein(s) with high sequence identity (>80% sequence identity), whole or immunogenic peptides to be used singly or in combination with other antigens in diagnostics/detection devices to detect antibodies which specifically bind to Rv1636 and which are present in a bodily fluid sample (serum, whole blood, plasma, saliva).

The disclosure provides isolated Rv3426 protein or protein(s) with high sequence identity (>80% sequence identity), whole or immunogenic peptides to be used singly or in combination with other antigens in diagnostics/detection devices to detect antibodies which specifically bind to Rv3426 and which are present in a bodily fluid sample (serum, whole blood, plasma, saliva).

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv0222 or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv3120 or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv1989c or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv3118 or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv1636 or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes the Rv3426 or protein(s) with high sequence identity (>80% sequence identity), full-length, or immunogenic fragments thereof is a native protein, conjugated to a molecule, or part of a complex of multiple polypeptide chains.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes a combination of the Rv0222 and/or Rv3120 and/or Rv1989c or protein(s) with highly similar sequence identity (>80% sequence identity), full-length, or immunogenic fragments, thereof is a native protein, conjugated to a molecule, or part of a complex of multiple polypeptide chains, with the following combinations Rv0222 and Rv3120, Rv0222 and Rv1989c, Rv3120 and Rv1989c, Rv0222 and Rv3120 and Rv1989c.

The disclosure provides a diagnostics tool such as a TB lateral flow rapid diagnostic test or an enzyme-linked immunoassay detecting interferon-γ (IFN-7) released by the T-cells or an enzyme-linked immunospot assay detecting IFN-γ-producing cells, and others that utilizes a combination of the Rv3118 and/or Rv1636 and/or Rv3426 or protein(s) with highly similar sequence identity (>80% sequence identity), full-length, or immunogenic fragments, thereof is a native protein, conjugated to a molecule, or part of a complex of multiple polypeptide chains, with the following combinations Rv3118 and Rv1636, Rv3118 and Rv3426, Rv1636 and Rv3426, Rv3118 and Rv1636 and Rv3426.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-γ-producing cells, and others that utilizes a combination of the Rv0222 and/or Rv3120 and/or Rv1989c and/or Rv3118 and/or Rv1636 and/or Rv3426 and/or Rv2185c and/or Rv3354 protein(s) with highly similar sequence identity (>80% sequence identity), full-length, or immunogenic fragments, thereof is a native protein, conjugated to a molecule, or part of a complex of multiple polypeptide chains. In some embodiments, the following combinations are used: Rv0222 and Rv3118; Rv0222 and Rv1636; Rv0222 and Rv3426; Rv0222 and Rv3354; Rv0222 and Rv2185c; Rv0222 and Rv3118 and Rv1636; Rv0222 and Rv3118 and Rv3426; Rv0222 and Rv1636 and Rv3426; Rv0222 and Rv3354 and Rv2185c; Rv1989c and Rv3118; Rv1989c and Rv1636; Rv1989c and Rv3426; Rv1989c and Rv3354; Rv1989c and Rv2185c; Rv1989c and Rv3118 and Rv1636; Rv1989c and Rv3118 and Rv3426; Rv1989c and Rv1636 and Rv3426; Rv1989c and Rv3354 and Rv2185c; Rv3120 and Rv3118; Rv3120 and Rv1636; Rv3120 and Rv3426; Rv3120 and Rv3354; Rv3120 and Rv2185c; Rv3120 and Rv3118 and Rv1636; Rv3120 and Rv3118 and Rv3426; Rv3120 and Rv1636 and Rv3426; Rv3120 and Rv3354 and Rv2185c; Rv0222 and Rv1989c and Rv3118; Rv0222 and Rv1989c and Rv1636; Rv0222 and Rv1989c and Rv3426; Rv0222 and Rv1989c and Rv3354; Rv0222 and Rv1989c and Rv2185c; Rv0222 and Rv1989c and Rv3118 and Rv1636; Rv0222 and Rv1989c and Rv3118 and Rv3426; Rv0222 and Rv1989c and Rv1636 and Rv3426; Rv0222 and Rv1989c and Rv3354 and Rv2185c; Rv0222 and Rv3120 and Rv3118; Rv0222 and Rv3120 and Rv1636; Rv0222 and Rv3120 and Rv3426; Rv0222 and Rv3120 and Rv3354; Rv0222 and Rv3120 and Rv2185c; Rv0222 and Rv3120 and Rv3118 and Rv1636; Rv0222 and Rv3120 and Rv3118 and Rv3426; Rv0222 and Rv3120 and Rv1636 and Rv3426; Rv022 and Rv3120 and Rv3354 and Rv2185c; Rv1989c and Rv3120 and Rv3118; Rv1989c and Rv3120 and Rv1636; Rv1989c and Rv3120 and Rv3426; Rv1989c and Rv3120 and Rv3354; Rv1989c and Rv3120 and Rv2185c; Rv1989c and Rv3120 and Rv3118 and Rv1636; Rv1989c and Rv3120 and Rv3118 and Rv3426; Rv1989c and Rv3120 and Rv1626 and Rv3426; Rv1989c and Rv3120 and Rv3354 and Rv2185c; Rv0222 and Rv1989c and Rv3120 and Rv3118, Rv0222 and Rv1989c and Rv3120 and Rv1636; Rv0222 and Rv1989c and Rv3120 and Rv3426; Rv0222 and Rv1989c and Rv3120 and Rv3354; Rv0222 and Rv1989c and Rv3120 and Rv2185c; Rv0222 and Rv1989c and Rv3120 and Rv3118 and Rv1636; Rv0222 and Rv1989c and Rv3120 and Rv3118 and Rv3426, Rv0222 and Rv1989c and Rv3120 and Rv1636 and Rv3426; Rv0222 and Rv1989c and Rv3120 and Rv3354 and Rv2185c; Rv0222 and Rv1989c and Rv3120 and Rv3118 and Rv1636 and Rv3426; and Rv0222 and Rv1989c and Rv3120 and Rv3354 and Rv2185c and Rv3118 and Rv1636 and Rv3426.

The disclosure provides a diagnostics tool such as a TB lateral flow rapid diagnostic test or an enzyme-linked immunoassay detecting interferon-γ (IFN-γ) released by the T-cells or an enzyme-linked immunospot assay detecting IFN-7-producing cells, and others that utilizes a combination of the Rv0222, and/or Rv1989c, and/or Rv3120, and/or Rv3118, and/or Rv1636, and/or Rv3426 and/or with other TB antigens. The preceding aspects of the disclosure are not claimed.

In one aspect, the invention provides a method of detecting antibodies in a sample from a subject, wherein the antibodies bind to epitopes of *Mycobacterium tuberculosis* antigens, comprising
i. contacting the sample with five polypeptides comprising SEQ ID NOS: 1, 3, 5, 7 and 9; and
ii. detecting formation of antibody-peptide complexes comprising said polypeptides;
wherein formation of said complexes is indicative of the presence of the antibodies to epitopes of *Mycobacterium tuberculosis* antigens in said sample. In some embodiments, the subject has been infected with *Mycobacterium tuberculosis.* In some embodiments, the polypeptides are linked to an affinity tag sequence to facilitate purification. In some embodiments, the affinity tag is a 6x-Histidine tag.

In another aspect, the invention provides a method for assaying interferon-γ production from a sample comprising T cells from a subject, comprising
i. contacting the sample comprising T cells from the subject with five polypeptides comprising SEQ ID NOS: 1, 3, 5, 7 and 9; and
ii. detecting the presence of interferon-γ produced by the T cells.

The disclosure provides a method, which is not claimed, for detecting interferon-γ producing T cells from a sample from a subject, comprising
i. contacting the sample comprising T cells from the subject with two or more isolated polypeptides or antigenic fragments or variants thereof, wherein the polypeptides comprise polypeptides selected from the group consisting of SEQ ID NOS: 1, 3, 5, 7, 9, 11, 13, and 15; and
ii. detecting the presence of interferon-γ producing T cells.

In some embodiments, the sample is incubated with the two or more isolated polypeptides or antigenic fragments or variants thereof for between 4 and 24 hours prior to detecting the presence of interferon-γ produced by the T cells. In some embodiments, the T cells are freshly isolated. The T cells may be in blood or isolated from blood. In some embodiments, the T cells comprise CD4+ and CD8+ T cells. In some embodiments, the T cells comprise CD4+ immediate effector T cells. In some embodiments, the subject has been infected with *Mycobacterium tuberculosis.* In some embodiments, the sample is selected from the group consisting of blood, serum, plasma, lymph nodes, skin, saliva, urine, cerebrospinal fluid and milk.

In another aspect, the invention provides a device, wherein the device comprises five polypeptides, wherein the polypeptides comprise SEQ ID NOS: 1, 3, 5, 7 and 9. In some embodiments, the five polypeptides are attached or immobilized to a solid support. In some embodiments, the two or more isolated polypeptides are attached or immobilized to a bead, a flow path in a lateral flow immunoassay device, a well in a microtiter plate, or a flow path in a rotor. In some embodiments, the device is for assaying for the presence of antibodies in a sample from a subject, wherein the antibodies bind to epitopes of *Mycobacterium tuberculosis* antigens.

### BRIEF DESCRIPTION OF THE FIGURES

The skilled artisan will understand that the drawings, described below, are for illustration purposes only. The drawings are not intended to limit the scope of the present teachings in any way.
FIG. 1. Schematics of a lateral-flow rapid test strip.
FIG. 2A. Examples of the single-path lateral flow TB test. A) Example of a housing cassette for the lateral flow test strip. B) Example of a 3-antigen lateral flow strip test after being assayed with TB-positive sample obtained from the World Health Organization. The 3 antigens used with this strip are Rv0222 (top line), Rv1989c (middle line), and Rv3120 (bottom line). C) Example of a 5-antigen test strip after assayed with a TB positive sample. D) Example of a lateral flow prototype before the assay. (Note: No control line in these lateral flow strip examples).
FIG. 2B. Examples of 5-antigen IgM-lateral flow test strips after assayed with TB positive/negative samples obtained from the World Health Organization. The 5 antigens used with these strips are Rv0222, Rv1989c, Rv3120, Rv3354, and Rv2185c. The TB samples are Sample #98 - Smear positive/Culture positive (Active TB); Sample #185 - Smear negative/Culture negative/Chest X-ray positive with improvement upon treatment (Active TB); Sample #105 - Smear negative/Culture negative (Non-active TB).
FIG. 3. A) Example of a dual-path lateral flow test. The device has been assayed with a serum sample, hence the control line is visible. B) The top part of the housing cassette. C) The low part of the cassette and the 2 lateral flow strips allowing the evaluation of up to 10 analytes in the sample. a) Sample pad, b) Conjugate pad, c) Chasing buffer reservoir.

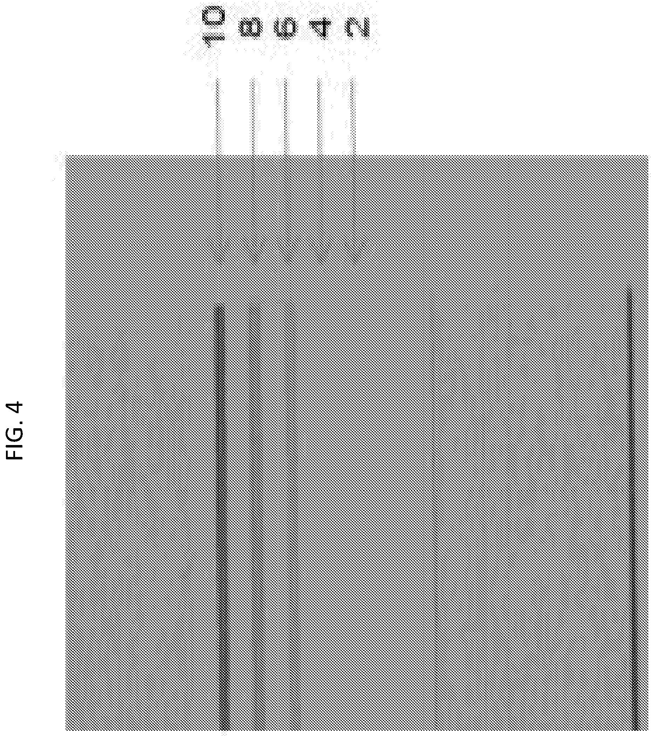

FIG. 4. A typical Rann scale to be used to rank signal intensity visualized by the naked eyes.

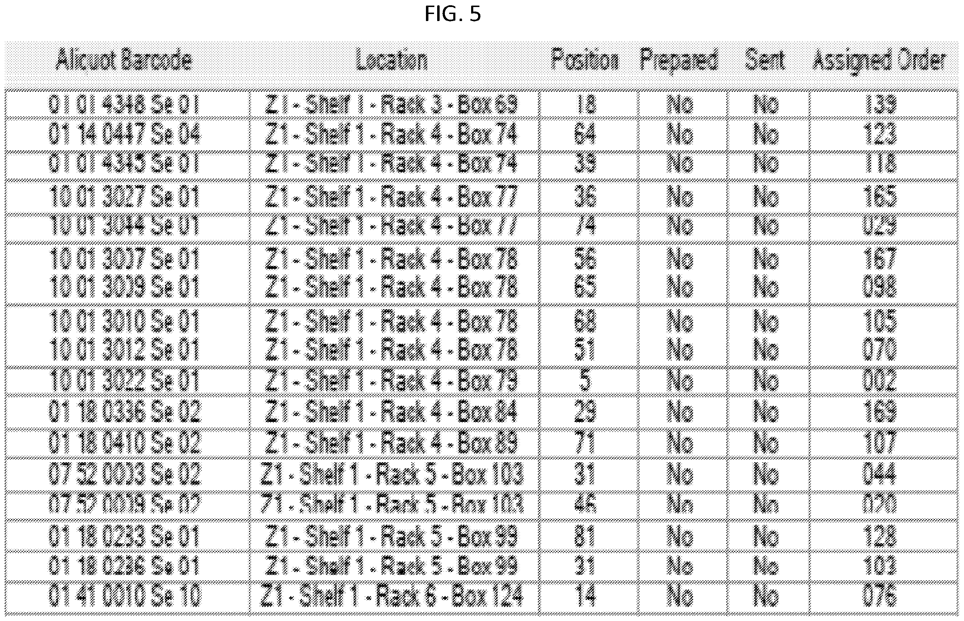

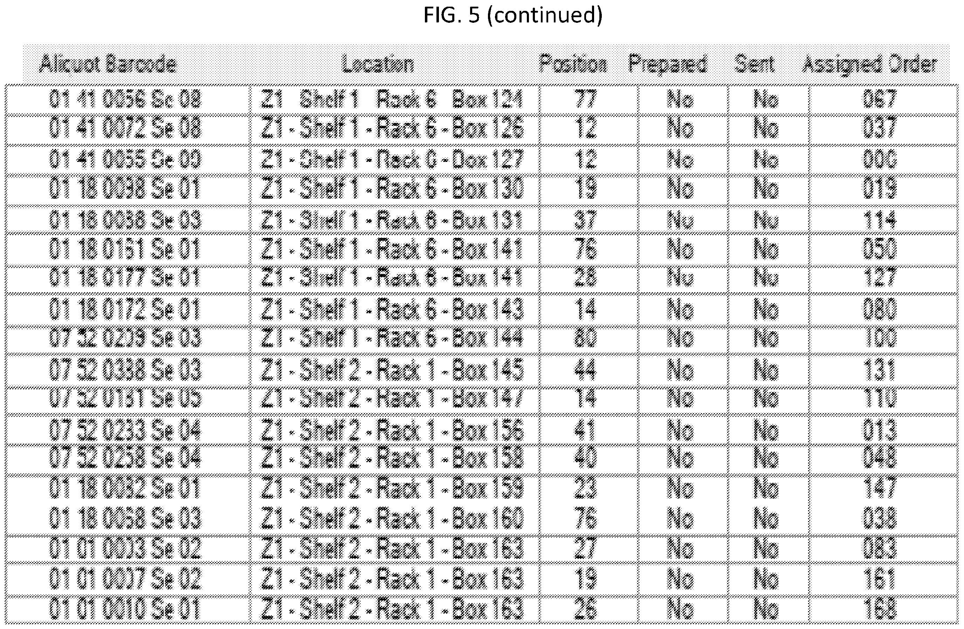

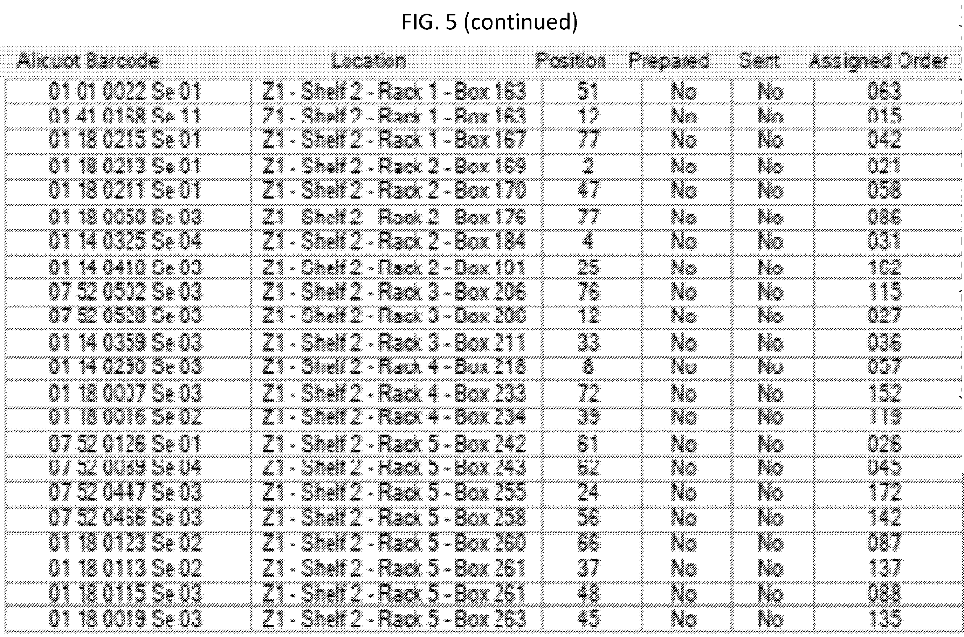

FIG. 5. Codes for the TB serum samples received from WHO/FIND.

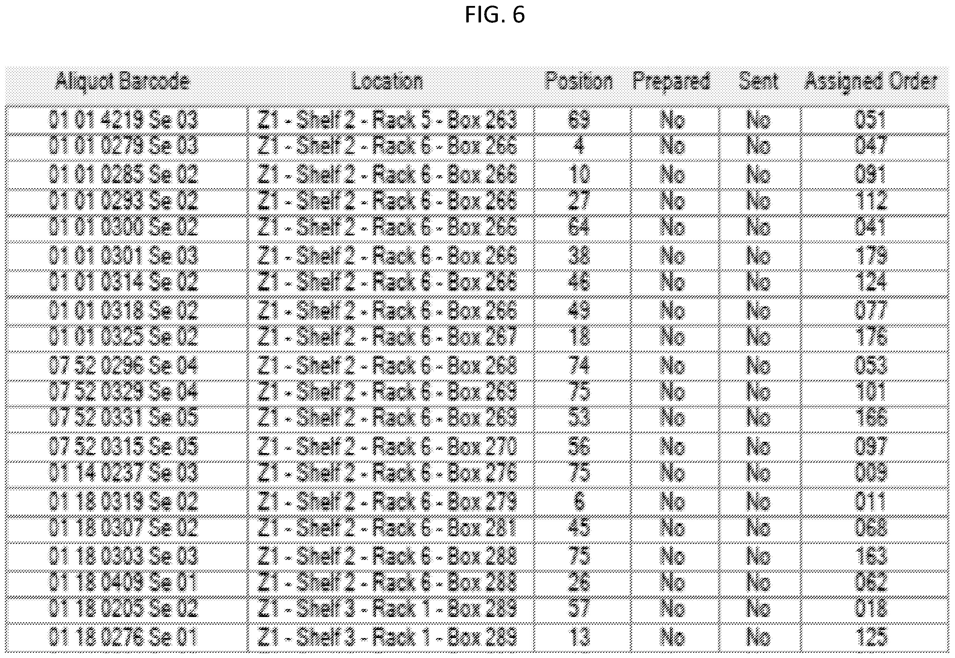

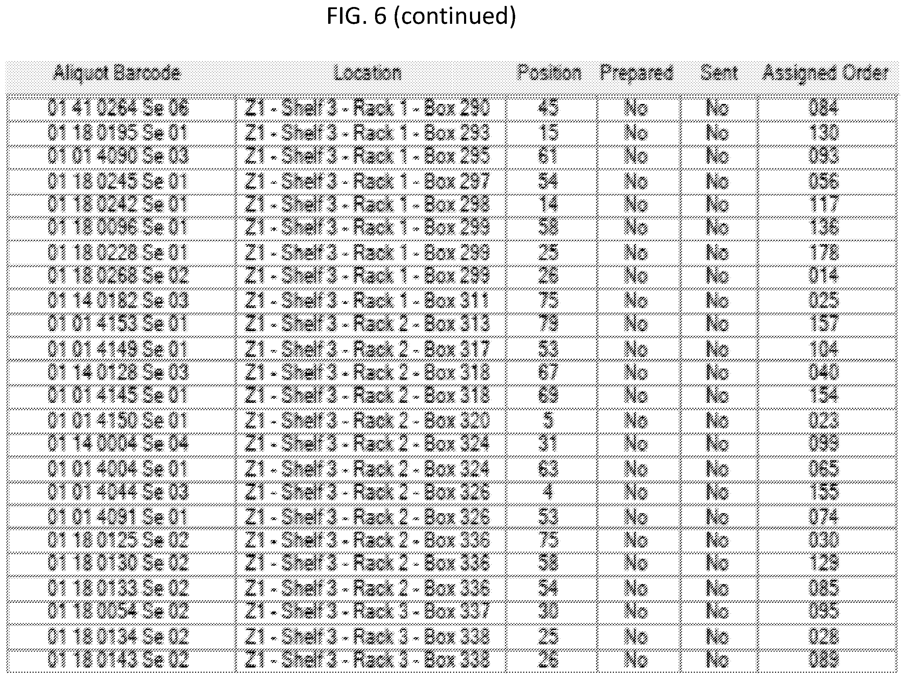

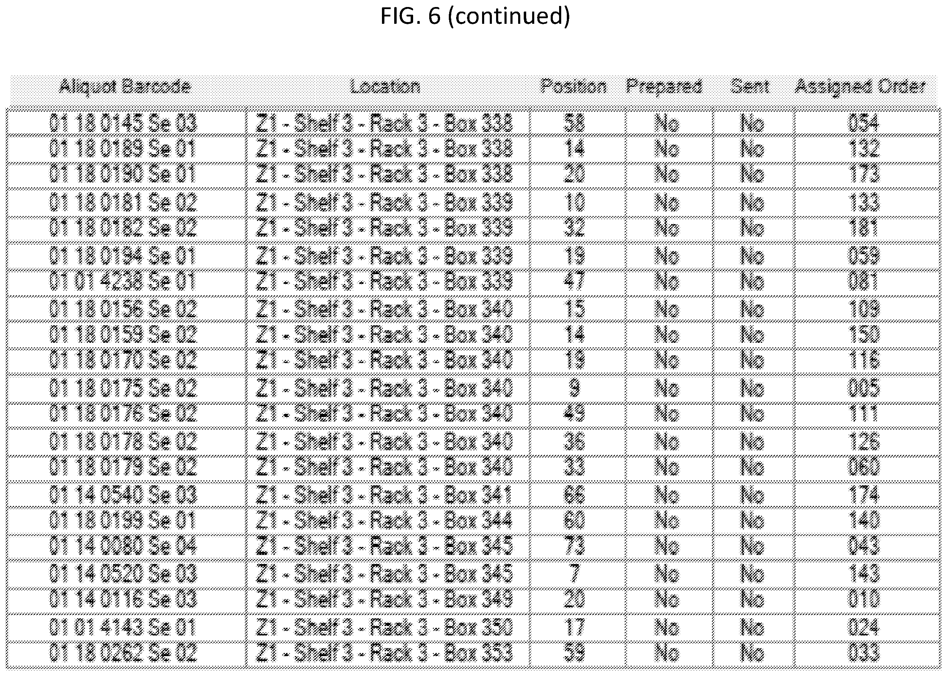

FIG. 6. Codes for the TB serum samples received from WHO/FIND.

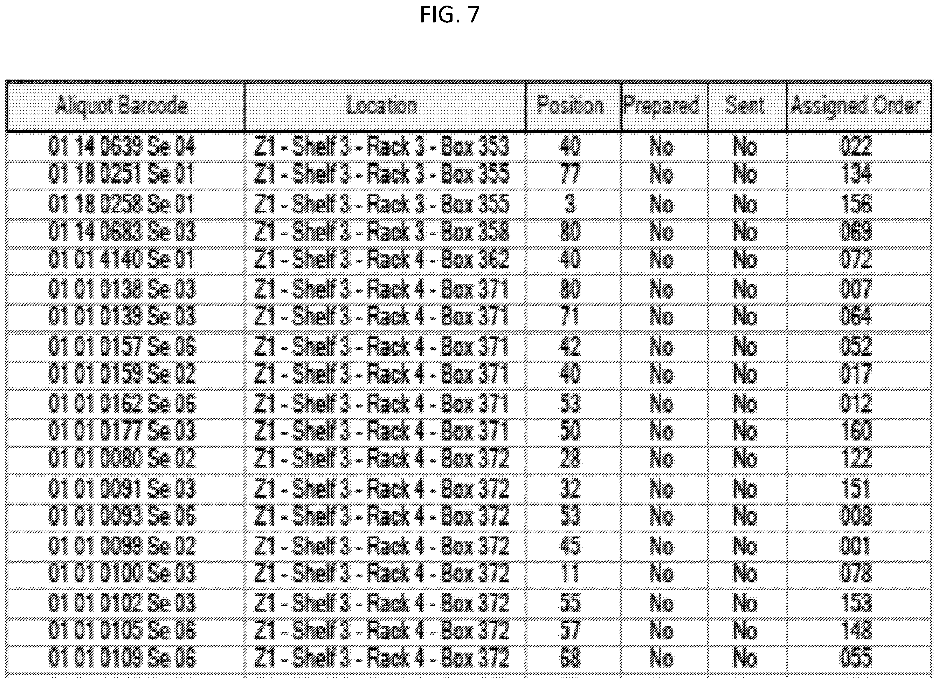

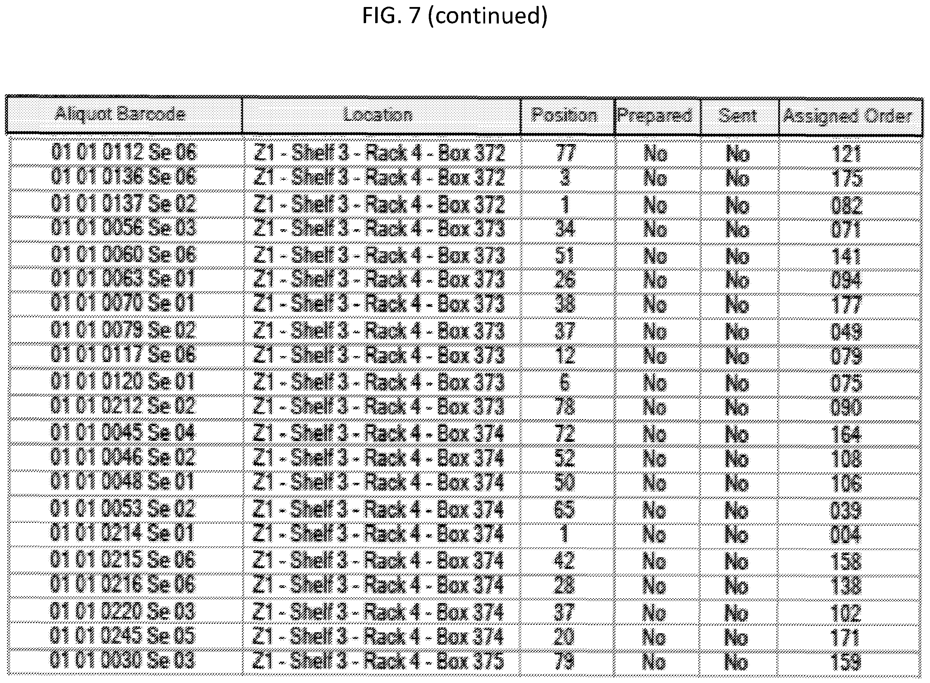

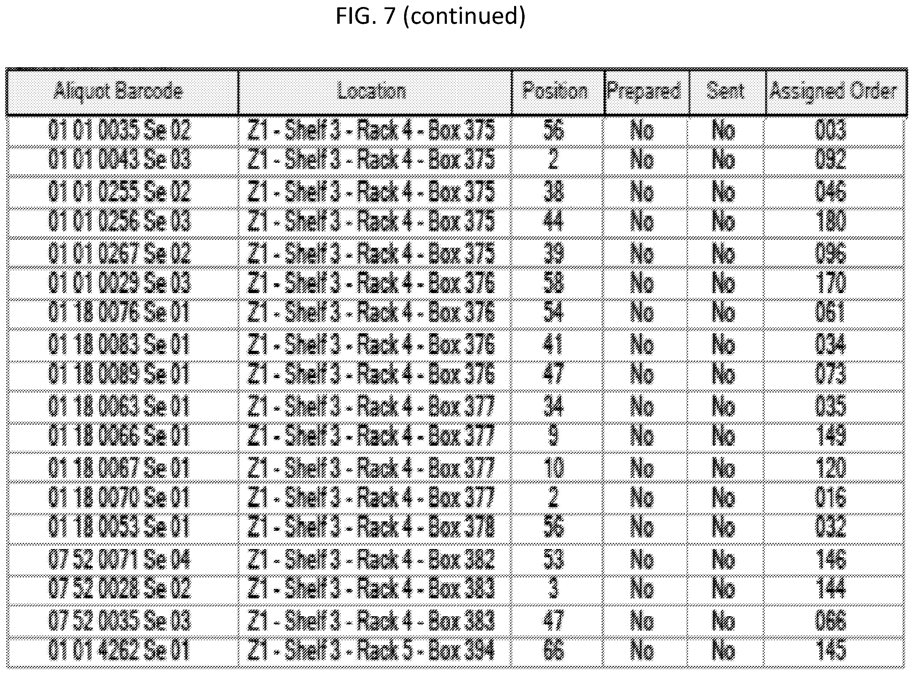

FIG. 7. Codes for the TB serum samples received from WHO/FIND.

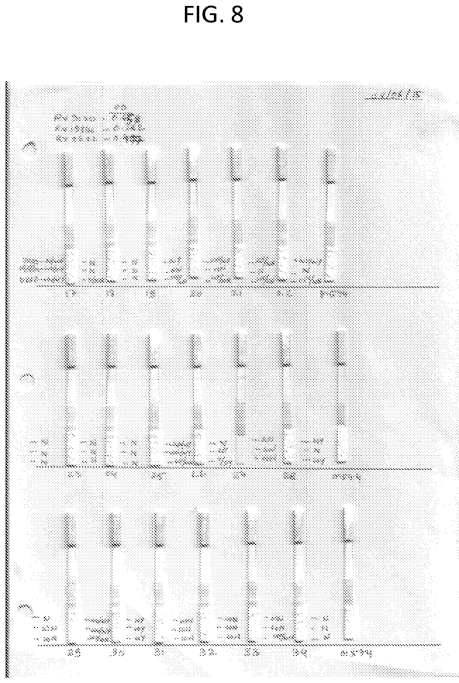

FIG. 8. Typical lateral flow IgM strips after assayed with WHO/FIND TB serum samples. The strips were made with the Rv0222, Rv1989c, Rv3120 antigens for IgM detection.

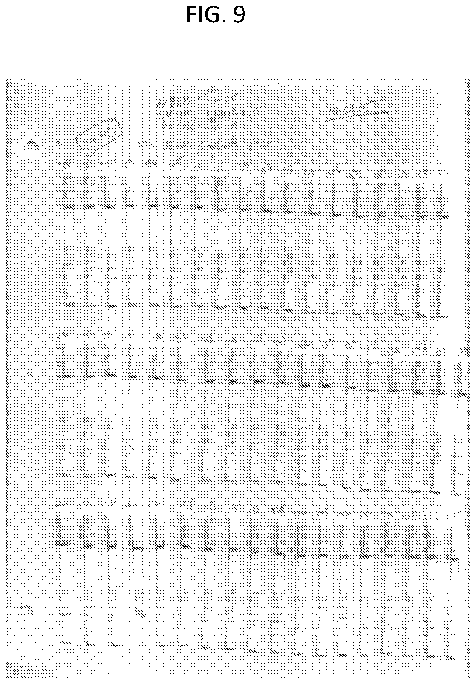

FIG. 9. Typical lateral flow IgG strips after assayed with WHO/FIND TB serum samples. The strips were made with the Rv0222, Rv1989c, Rv3120 antigens for IgG detection.

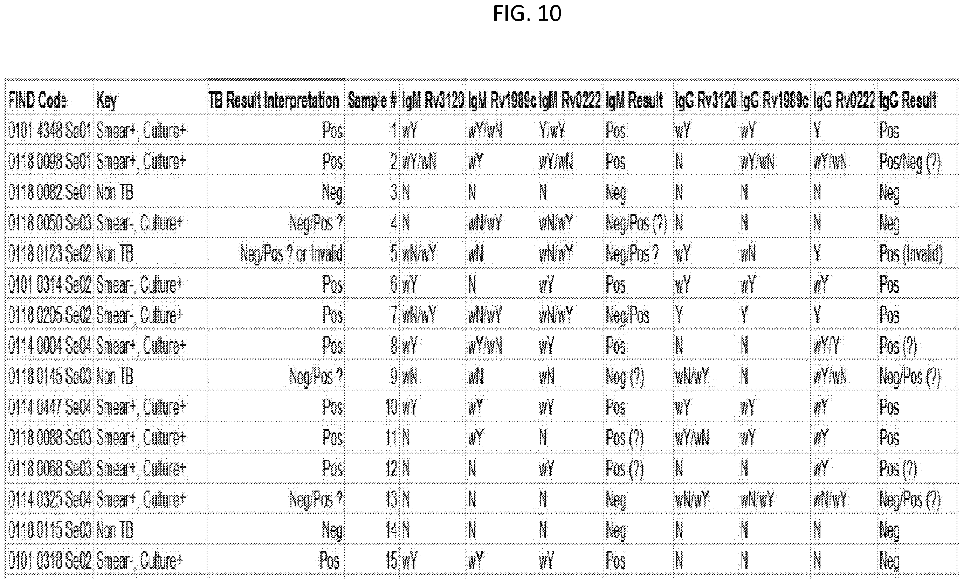

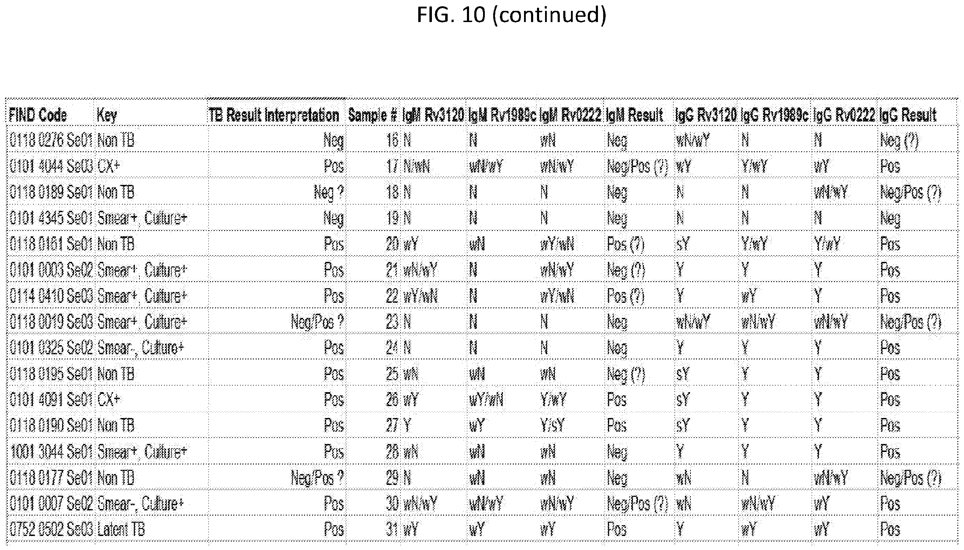

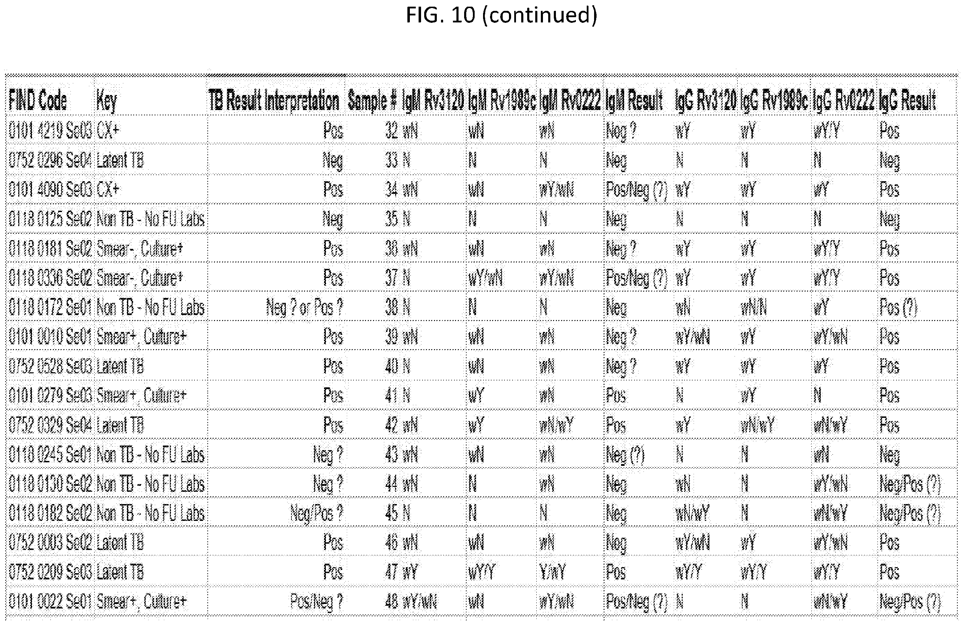

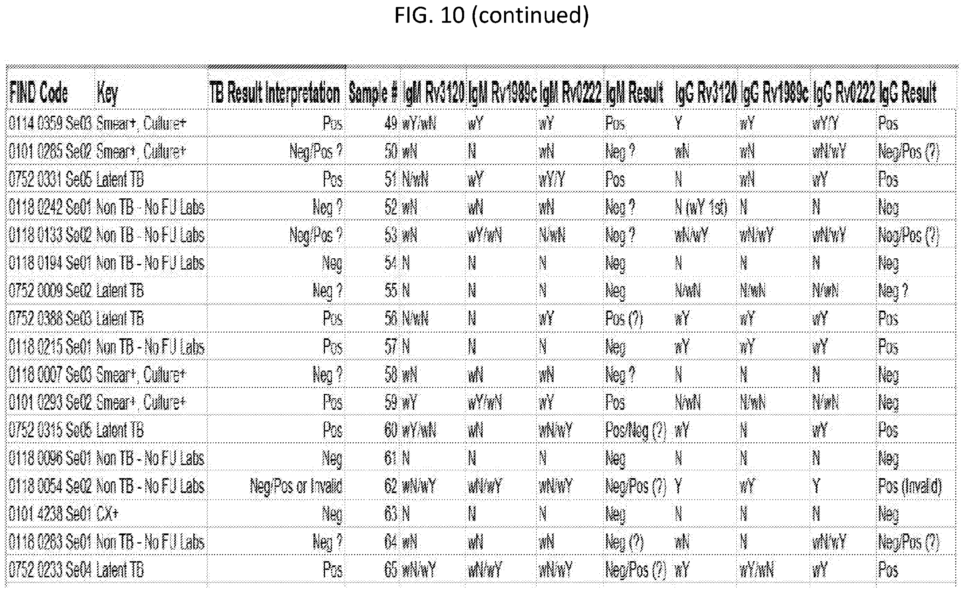

FIG. 10. Summary of lateral flow test results after assaying with the WHO/FIND TB samples using strips made with the Rv0222, Rv1989c, Rv3120 antigens.

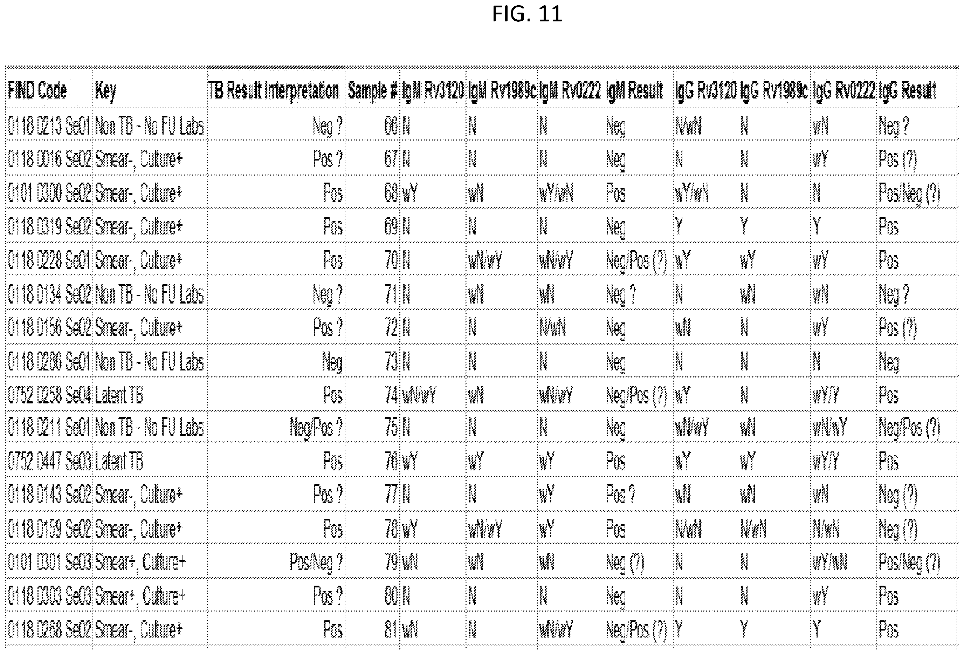

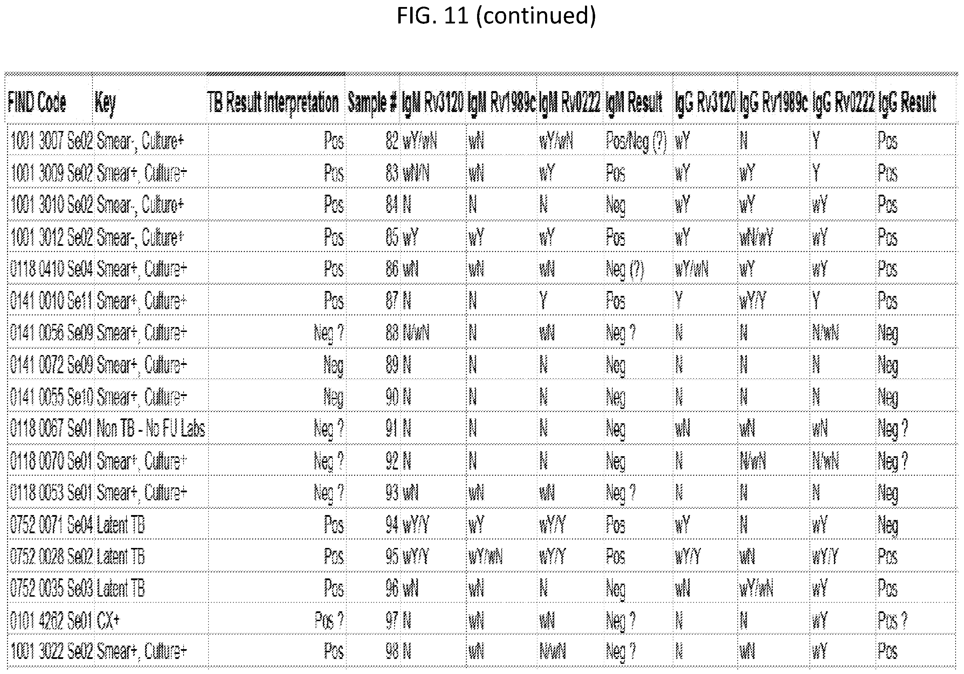

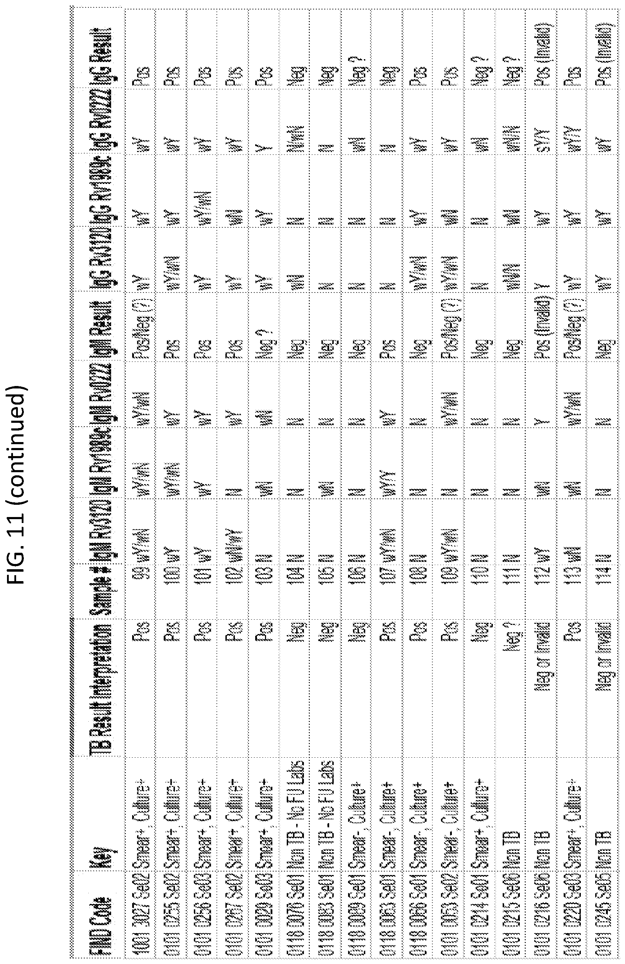

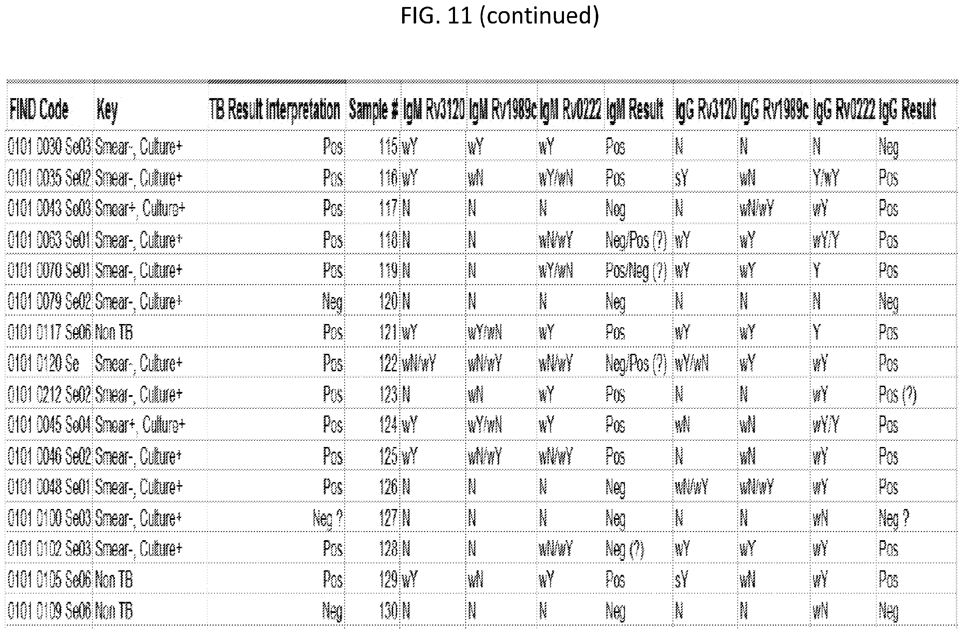

FIG. 11. Summary of lateral flow test results after assaying with the WHO/FIND TB samples using strips made with the Rv0222, Rv1989c, Rv3120 antigens.

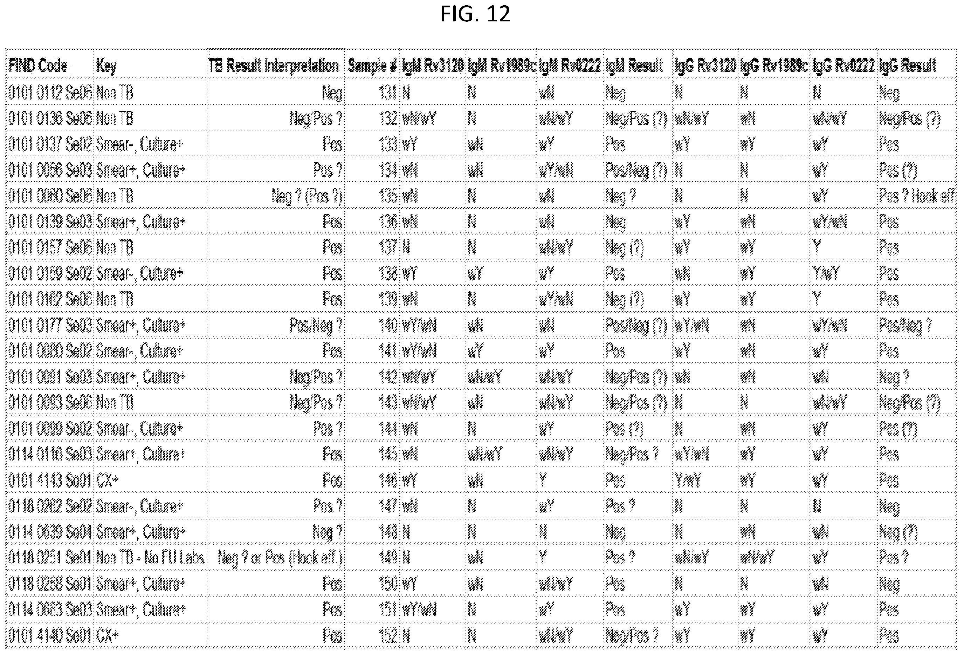

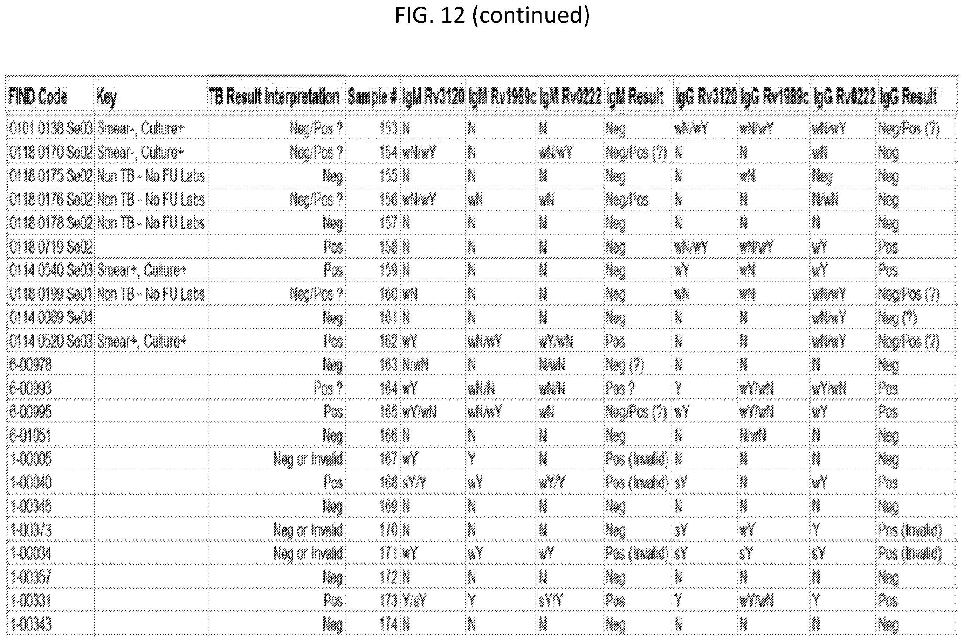

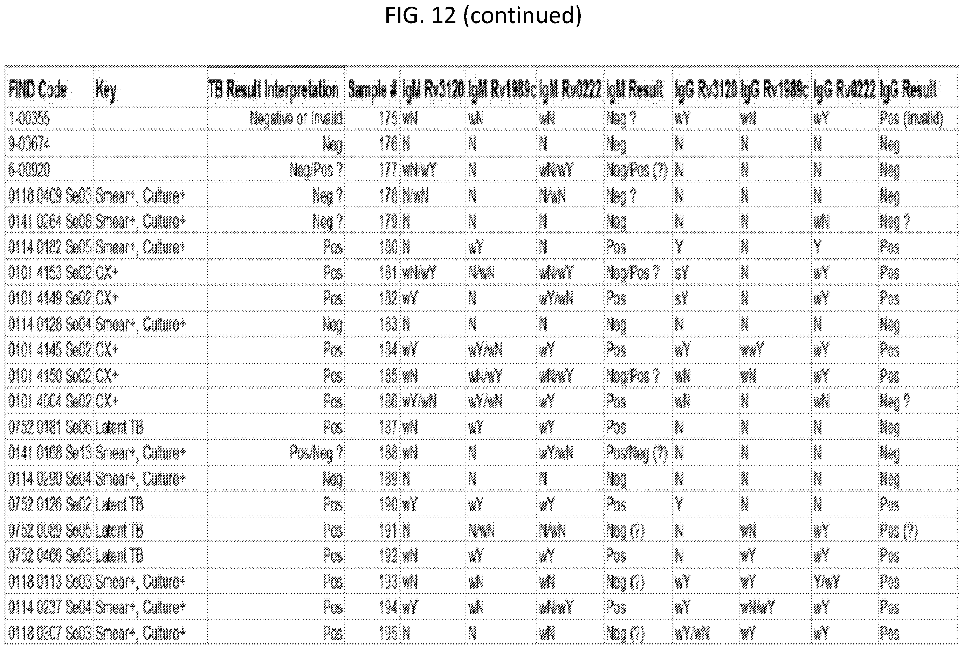

FIG. 12. Summary of lateral flow test results after assaying with the WHO/FIND TB samples using strips made with the Rv0222, Rv1989c, Rv3120 antigens.

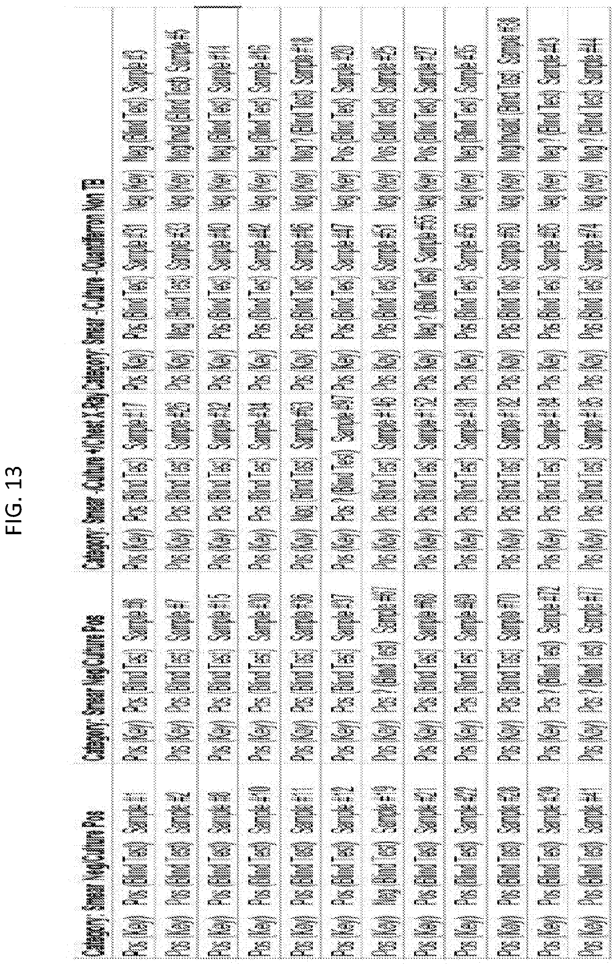

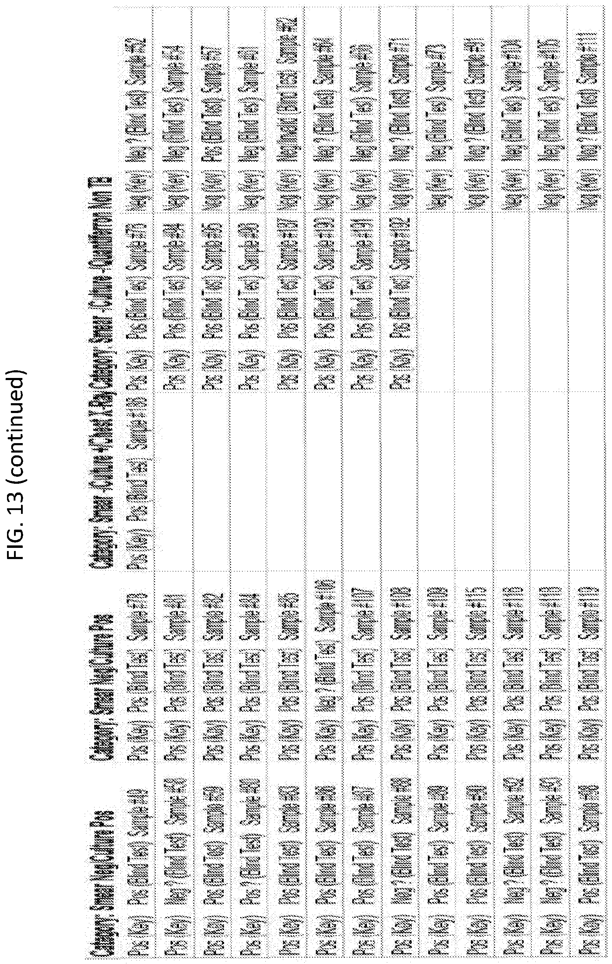

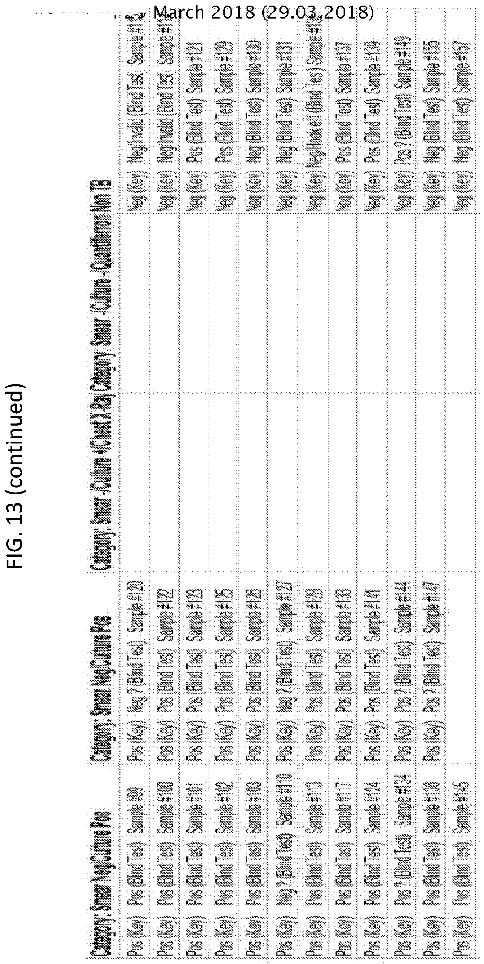

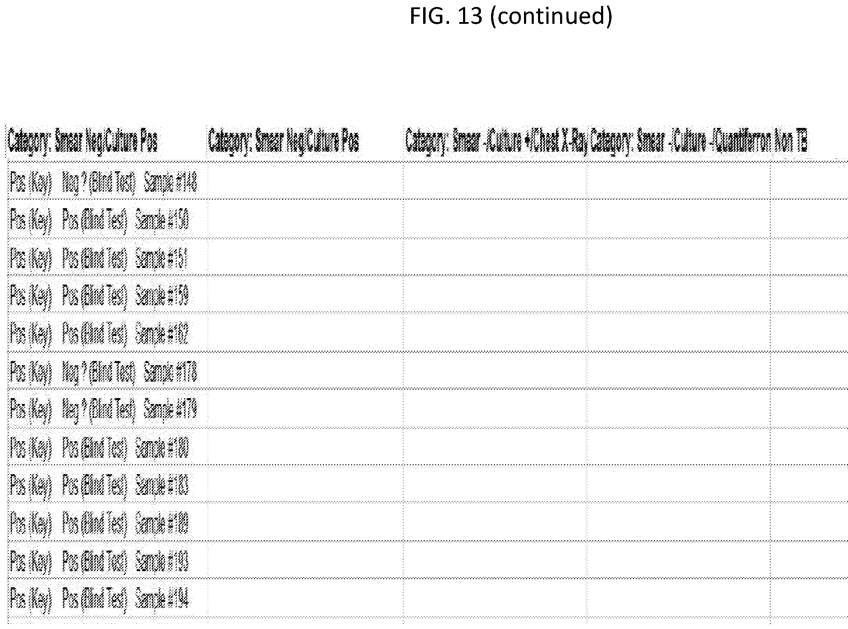

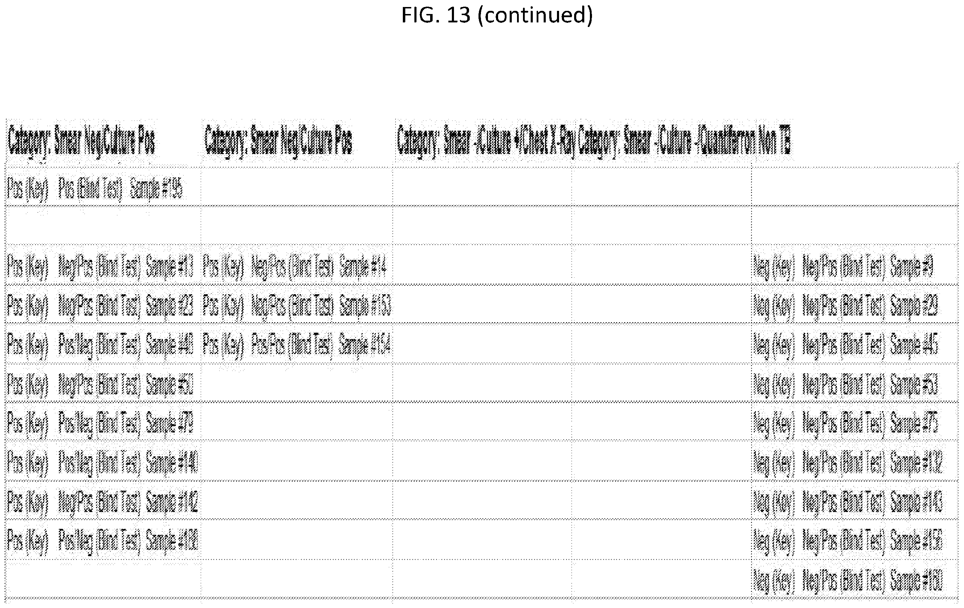

FIG. 13. Tabulation of lateral flow test result interpretation after assaying with the WHO/FIND TB samples using strips made with the Rv0222, Rv1989c, Rv3120 antigens.

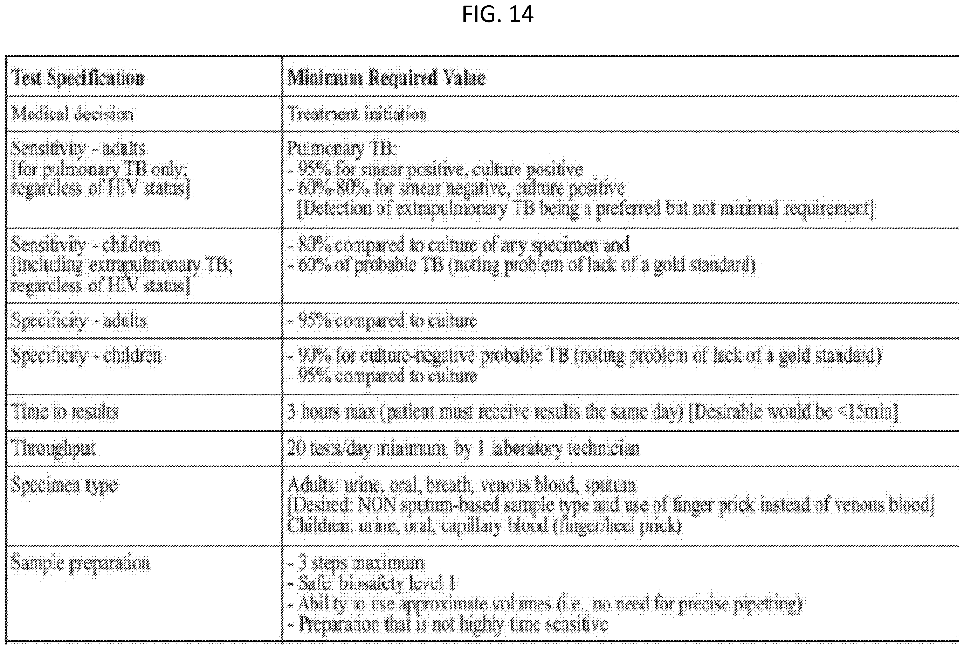

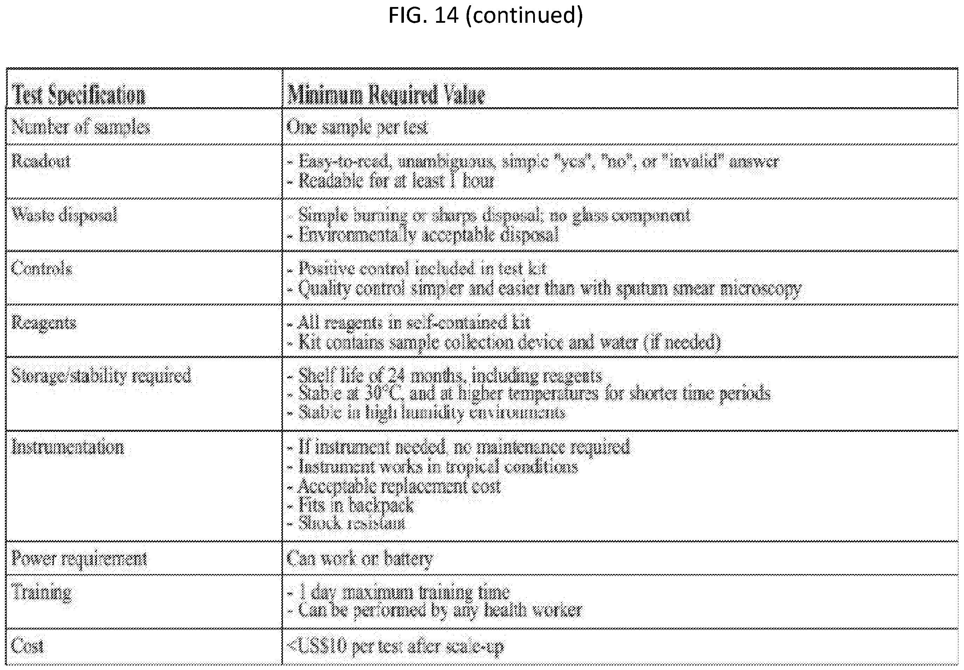

FIG. 14. The Minimum Specifications for a Point-Of-Care TB Test.

### DETAILED DESCRIPTION OF THE INVENTION

The present invention is based, in part, on the discovery that a combination of polypeptides from *Mycobacterium tuberculosis,* or antigenic fragments or variants thereof provide for robust detection of immune system responses - an antibody response or T-cell response against *Mycobacterium tuberculosis* infection. In some embodiments, the invention provides an assay and device having the appropriate sensitivity and specificity to detect tuberculosis-specific antibodies in patient samples and having significant advantages over currently available tests. Accordingly, in some embodiments, the invention provides compositions, devices, methods, and kits useful for the detection of antibodies that bind to *Mycobacterium tuberculosis* antigens and the diagnosis of tuberculosis in subjects.

Reference will now be made in detail to the presently preferred embodiments of the invention which, together with the drawings and the following examples, serve to explain the principles of the invention. These embodiments describe in sufficient detail to enable those skilled in the art to practice the invention. Unless defined otherwise, all technical and scientific terms used herein have the same meanings as commonly understood by one of ordinary skill in the art.

In some embodiments, the practice of the present invention employs various techniques of molecular biology (including recombinant techniques), microbiology, cell biology, biochemistry and immunology. *See, e.g.,* Sambrook et al. Molecular Cloning: A Laboratory Manual, 2nd edition (1989); Current Protocols in Molecular Biology (F. M. Ausubel et al. eds. (1987)); the series Methods in Enzymology (Academic Press, Inc.); PCR: A Practical Approach (M. MacPherson et al. IRL Press at Oxford University Press (1991)); PCR 2: A Practical Approach (M. J. MacPherson, B. D. Hames and G. R. Taylor eds. (1995)); Antibodies, A Laboratory Manual (Harlow and Lane eds. (1988)); Using Antibodies, A Laboratory Manual (Harlow and Lane eds. (1999)); and Animal Cell Culture (R. I. Freshney ed. (1987)).

Definitions of common terms in molecular biology may be found, for example, in Benjamin Lewin, Genes VII, published by Oxford University Press, 2000 (ISBN 019879276X); Kendrew et al. (eds.); The Encyclopedia of Molecular Biology, published by Blackwell Publishers, 1994 (ISBN 0632021829); and Robert A. Meyers (ed.), Molecular Biology and Biotechnology: a Comprehensive Desk Reference, published by Wiley, John & Sons, Inc., 1995 (ISBN 0471186341).

For the purpose of interpreting this specification, the following definitions will apply and whenever appropriate, terms used in the singular will also include the plural and vice versa. In the event that any definition set forth below conflicts with the usage of that word in any other document, the definition set forth below shall always control for purposes of interpreting this specification and its associated claims unless a contrary meaning is clearly intended (for example in the document where the term is originally used). The use of "or" means "and/or" unless stated otherwise. As used in the specification and claims, the singular form "a," "an" and "the" include plural references unless the context clearly dictates otherwise. For example, the term "an antibody" includes a plurality of antibodies, including mixtures thereof. The use of "comprise," "comprises," "comprising," "include," "includes," and "including" are interchangeable and not intended to be limiting. Furthermore, where the description of one or more embodiments uses the term "comprising," those skilled in the art would understand that, in some specific instances, the embodiment or embodiments can be alternatively described using the language "consisting essentially of" and/or "consisting of."

As used herein, the term "about" means plus or minus 10% of the numerical value of the number with which it is being used.

The term "antigen," as used herein, refers to a molecule capable of being recognized by an antibody. An antigen can be, for example, a peptide or a modified form thereof. An antigen can comprise one or more epitopes.

The term "epitope," as used herein, is a portion of an antigen that is specifically recognized by an antibody. An epitope, for example, can comprise or consist of a portion of a peptide (e.g., a peptide of the invention). An epitope can be a linear epitope, sequential epitope, or a conformational epitope. In certain embodiments, epitopes may comprise noncontiguous regions.

The terms "nucleic acid," "oligonucleotide" and "polynucleotide" are used interchangeably herein and encompass DNA, RNA, cDNA, whether single stranded or double stranded, as well as chemical modifications thereof.

Single letter amino acid abbreviations used herein have their standard meaning in the art, and all peptide sequences described herein are written according to convention, with the N-terminal end to the left and the C-terminal end to the right.

### Methods

In one embodiment, the invention provides a method of detecting antibodies in a sample from a subject, wherein the antibodies bind to epitopes of *Mycobacterium tuberculosis* antigens, comprising
i. contacting the sample with five polypeptides comprising SEQ ID NOS: 1, 3, 5, 7 and 9; and
ii. detecting formation of antibody-peptide complexes comprising said isolated polypeptides;
wherein formation of said complexes is indicative of the presence of the antibodies to epitopes of *Mycobacterium tuberculosis* antigens in said sample.

The sample is not particularly limiting. In some embodiments, the sample is selected from the group consisting of blood, serum, plasma, lymph nodes, skin, saliva, urine, cerebrospinal fluid and milk.

The *Mycobacterium tuberculosis* antigens comprise polypeptides comprising SEQ ID NOS: 1, 3, 5, 7 and 9. The amino acid and nucleic acid sequences of the *Mycobacterium tuberculosis* antigens are shown below.

### Rv2185c

Amino Acid: 
MADKTTQTIYIDADPGEVMKAIADIEAYPQWISEYKEVEILEADDEGYPKRARML
MDAAIFKDTLIMSYEWPEDRQSLSWTLESSSLLKSLEGTYRLAPKGSGTEVTYEL
AVDLAVPMIGMLKRKAERRLIDGALKDLKKRVEG (SEQ ID NO:1)
Nucleotide (Strain H37Rv):

gtggcggaca agacgacaca gacgatttac atcgacgcgg atccaggcga ggtgatgaag gcgatcgccg
acatcgaagc ctacccgcaa tggatttcgg agtataagga agtcgagatc ctagaggccg acgacgaggg
ctacccgaaa cgagcgcgaa tgttgatgga cgcagccatc ttcaaagaca ccttgatcat gtcctacgag tggccggaag
accgccaatc gcttagctgg actctcgaat ccagctcgct gctaaagtcc ctcgaaggca cgtatcgctt ggcgcccaag
ggttctggca ctgaggtcac ctacgagctt gccgtcgacc ttgctgtccc catgatcggg atgctcaagc gtaaggcgga
acgcaggttg atagacggcg cgttgaagga tctgaagaaa cgagtcgagg gctga (SEQ ID NO:2)

### Rv3354

Amino Acid: 
MNLRRHQTLTLRLLAASAGILSAAAFAAPAQANPVDDAFIAALNNAGVNYGDP
VDAKALGQSVCPILAEPGGSFNTAVASVVARAQGMSQDMAQTFTSIAISMYCPS
VMADVASGNLPALPDMPGLPGS (SEQ ID NO:3)
Nucleotide (Strain H37Rv):

atgaacctac ggcgccatca gaccctgacg ctgcgactgc tggcggcatc cgcgggcatt ctcagcgccg
cggccttcgc cgcgccagca caggcaaacc ccgtcgacga cgcgttcatc gccgcgctga acaatgccgg
cgtcaactac ggcgatccgg tcgacgccaa agcgctgggt cagtccgtct gcccgatcct ggccgagccc
ggcgggtcgt ttaacaccgc ggtagccagc gttgtggcgc gcgcccaagg catgtcccag gacatggcgc

aaaccttcac cagtatcgcg atttcgatgt actgcccctc ggtgatggca gacgtcgcca gcggcaacct gccggccctg
ccagacatgc cggggctgcc cgggtcctag (SEQ ID NO:4)

### Rv0222 (or echA1 - enoyl-CoA hydratase EchA1)

Amino Acid: 
MSSESDAANTEPEVLVEQRDRILIITINRPKAKNAVNAAVSRGLADAMDQLDGD
AGLSVAILTGGGGSFCAGMDLKAFARGENVVVEGRGLGFTERPPTKPLIAAVEG
YALAGGTELALAADLIVAARDSAFGIPEVKRGLVAGGGGLLRLPERIPYAIAMEL
ALTGDNLPAERAHELGLVNVLAEPGTALDAAIALAEKITANGPLAVVATKRIITE
SRGWSPDTMFAEQMKILVPVFTSNDAKEGAIAFAERRRPRWTGT (SEQ ID NO:5)
Nucleotide (Strain H37Rv):

atgagcagcg aaagcgacgc agccaacacc gaacctgagg ttctggtaga acagcgggat cggattttga tcatcacgat
caaccgcccg aaagccaaga acgcggtcaa cgccgcagtc agccggggct tggccgatgc gatggatcag
cttgacggcg atgccggcct gtcggtggca atcctgaccg gtgggggcgg ttcgttctgc gcgggcatgg acctcaaggc
gttcgcccgg ggcgagaatg tcgtcgtcga aggtcgcggc cttggcttta ccgaacgtcc gccgaccaag ccgctcattg
ctgcggtgga aggctacgcg ttggcgggtg gcaccgagct ggcgcttgct gccgacctga tcgtggcggc
cagggattcg gcgttcggga ttcctgaagt caagcggggt ctggttgccg gcggcggggg attgctgcgg
ttgccggagc gcatcccgta tgcgatagcc atggagttgg cgctgaccgg tgacaaccta ccggccgaac
gcgcgcacga gctggggctc gtcaacgttt tggccgagcc ggggaccgcc ctcgatgctg cgatcgcgtt
ggcggagaag atcaccgcca atgggccgct ggcggtggtg gccaccaagc ggattatcac cgagtcgcgt
gggtggagtc ccgacactat gttcgctgag cagatgaaga tcctggtgcc ggtgttcacc tccaacgacg cgaaggaagg
tgcgatcgcg ttcgccgaga ggcgccggcc ccgttggacg ggcacctag (SEQ ID NO:6)

### Rv1989c

Amino Acid: 
MSDALDEGLVQRIDARGTIEWSETCYRYTGAHRDALSGEGARRFGGRWNPPLLF
PAIYLADSAQACMVEVERAAQAASTTAEKMLEAAYRLHTIDVTDLAVLDLTTPQ
AREAVGLENDDIYGDDWSGCQAVGHAAWFLHMQGVLVPAAGGVGLVVTAYE
QRTRPGQLQLRQSVDLTPALYQELRAT (SEQ ID NO:7)
Nucleotide (Strain H37Rv):

gtgagcgatg ccctcgatga agggctcgtc cagcgtatcg acgcacgcgg aacaattgag tggtcggaaa
cgtgctaccg gtataccggc gcgcaccgtg acgccttgtc cggtgagggc gcgcgcagat tcggaggcag
gtggaatccg ccgctgctct ttccggcgat ctatcttgct gattccgccc aagcctgcat ggttgaggtg gaacgggcgg
cgcaagcggc ttcaacgacc gcagagaaga tgctcgaggc ggcctaccga ctacacacga tcgacgtcac
ggacctggcc gtcctcgatc tgacaacccc gcaagctcgg gaagccgtgg ggctcgagaa cgacgacatc
tatggcgacg actggtcagg gtgccaggcg gtcggacatg cggcctggtt cttgcacatg caaggtgtcc tcgtgccggc
ggcgggcggt gtcggcctcg ttgtcaccgc gtatgaacag cgaactcggc cgggccaact acaactgcga
caaagcgtcg atctgacgcc tgctctttac caagaacttc gagccacgta g (SEQ ID NO:8)

### Rv3120

Amino Acid: 
MSPSPSALLADHPDRIRWNAKYECADPTEAVFAPISWLGDVLQFGVPEGPVLEL
ACGRSGTALGLAAAGRCVTAIDVSDTALVQLELEATRRELADRLTLVHADLCSW
QSGDGRFALVLCRLFWHPPTFRQACEAVAPGGVVAWEAWRRPIDVARDTRRAE
WCLKPGQPESELPAGFTVIRVVDTDGSEPSRRIIAQRSL (SEQ ID NO:9)
Nucleotide (Strain H37Rv):

atgagtccgt ctccatcggc cctgctcgcc gaccacccgg accgcattcg ttggaacgcg aaatacgagt gcgctgaccc
cacggaggcg gtatttgcgc ccatatcctg gctcggcgac gtgctgcagt tcggggtgcc agaagggccg gttctggaac
tggcgtgcgg tcggtccggc accgcgctgg ggctagccgc ggcgggccgc tgcgtgactg cgatcgacgt
ttccgatacc gcgttggttc agctcgagct cgaagcgacc cgacgggaat tggccgatcg cctcacactg gtgcacgccg
atctctgctc ctggcagtcg ggggatggac gctttgctct ggtactttgc cgactattct ggcatccgcc cacttttcgc
caggcttgcg aggctgtggc gccgggcggt gtagtggcgt gggaggcatg gcggcggccc atcgatgtcg
ctcgggatac ccgtcgagcc gaatggtgct tgaagccagg ccagcccgag tctgaacttc ccgccggctt cacggtgatt
cgggtggtcg acaccgatgg ttcagagccg tcgcggcgca tcatcgccca acggtcactg tga (SEQ ID NO:10)

### Rv3118

Amino Acid: 
MCSGPKQGLTLPASVDLEKETVITGRVVDGDGQAVGGAFVRLLDSSDEFTAEVV
ASATGDFRFFAAPGSWTLRALSAAGNGDAVVQPSGAGIHEVDVKIT (SEQ ID
NO:11)
Nucleotide (Strain H37Rv):

atgtgctctg gacccaagca aggactgaca ttgccggcca gcgtcgacct ggaaaaagaa acggtgatca
ccggccgcgt agtggacggt gacggccagg ccgtgggcgg cgcgttcgtg cggctgctgg actcctccga
cgagttcacc gcggaggtcg tcgcgtcggc caccggcgat ttccggttct tcgccgcgcc cggatcctgg acgctgcgcg
cgctgtcggc ggccggcaac ggcgacgcgg tggtgcagcc ctcgggcgcg ggcatccacg aggtagacgt
caagatcacc tga (SEQ ID NO:12)

### Rv1636 (TB15.3)

Amino Acid: 
MSAYKTVVVGTDGSDSSMRAVDRAAQIAGADAKLIIASAYLPQHEDARAADILK
DESYKVTGTAPIYEILHDAKERAHNAGAKNVEERPIVGAPVDALVNLADEEKAD
LLVVGNVGLSTIAGRLLGSVPANVSRRAKVDVLIVHTT (SEQ ID NO:13)
Nucleotide (Strain H37Rv):

atgagcgcct ataagaccgt ggtggtagga accgacggtt cggactcgtc gatgcgagcg gtagatcgcg ctgcccagat
cgccggcgca gacgccaagt tgatcatcgc ctcggcatac ctacctcagc acgaggacgc tcgcgccgcc
gacattctga aggacgaaag ctacaaggtg acgggcaccg ccccgatcta cgagatcttg cacgacgcca
aggaacgagc gcacaacgcc ggtgcgaaaa acgtcgagga acggccgatc gtcggcgccc cggtcgacgc
gttggtgaac ctggccgatg aggagaaggc ggacctgctg gtcgtcggca atgtcggtct gagcacgatc
gcgggtcggc tgctcggatc ggtaccggcc aatgtgtcac gccgggccaa ggtcgacgtg ctgatcgtgc
acaccaccta g (SEQ ID NO:14)

### Rv3426 (PPE58)

Amino Acid: 
MHLMIPAEYISNVIYEGPRADSLYAADQRLRQLADSVRTTAESLNTTLDELHEN
WKGSSSEWMADAALRYLDWLSKHSRQILRTARVIESLVMAYEETLLRVVPPATI
ANNREEVRRLIASNVAGGKHSSNRRPRGTIRAVPGRKYPSNGPLSKLDPICAIEAA
PMAGAAADPQERVGPRGRRGLAGQQQCRGRPGPSLRCSHDTPRFQMNQAFHT
MVNMLLTCFACQEKPR (SEQ ID NO:15)
Nucleotide (Strain H37Rv):

atgcatctaa tgatacccgc ggagtatatc tccaacgtaa tatatgaagg tccgcgtgct gactcattgt atgccgccga
ccagcgattg cgacaattag ctgactcagt tagaacgact gccgagtcgc tcaacaccac gctcgacgag ctgcacgaga
actggaaagg tagttcatcg gaatggatgg ccgacgcggc tttgcggtat ctcgactggc tgtctaaaca ctcccgtcag

attttgcgaa ccgcccgcgt gatcgaatcc ctcgtaatgg cctatgagga gacacttctg agggtggtac ccccggcgac
tatcgccaac aaccgcgagg aggtgcgcag gctgatcgcg agcaacgtgg ccgggggtaa acactccagc
aatcgcagac ctcgaggcac aatacgagca gtaccgggcc gaaaatatcc aagcaatgga ccgctatcta agttggaccc
gatttgcgct atcgaagctg ccccgatggc gggagccgcc gcagatccac aggagcgggt aggtccaaga
ggccggcgcg gtcttgcagg ccagcaacaa tgccgcggtc gaccaggccc atcgcttcgc tgctcgcacg
acacaccgcg gtttcagatg aatcaggcgt ttcacaccat ggtgaacatg ttgctgacgt gttttgcatg tcaggagaaa
ccgagatga (SEQ ID NO:16).

In accordance with the method, the sample is contacted with five polypeptides, wherein the polypeptides comprise SEQ ID NOS: 1, 3, 5, 7 and 9.

The *Mycobacterium tuberculosis* polypeptides can be prepared in any suitable manner. Such polypeptides include isolated naturally occurring polypeptides, recombinantly produced polypeptides, synthetically produced polypeptides, or polypeptides produced by a combination of these methods.

In some embodiments, *the Mycobacterium tuberculosis* polypeptides are optimized for high level expression in *E. coli* using codons that are preferred in *E. coli.* In some embodiments, engineered antigenic fragments of *Mycobacterium tuberculosis* polypeptides (nucleic acid and amino acid sequences), which are optimized for expression in *E. coli* can be used, and which harbor an affinity tag (e.g., a histidine tag) to facilitate purification of the protein. As used herein, a codon that is "optimized for high level expression in *E. coli"* refers to a codon that is relatively more abundant in *E*. *coli* in comparison with all other codons corresponding to the same amino acid. In some embodiments, at least 40% of the codons are optimized for high level expression in *E*. *coli.* In some embodiments, at least 50%, at least 60%, at least 70%, at least 80%, at least 85%, at least 90%, at least 95%, or at least 99% of the codons are optimized for high level expression in *E. coli.* In some embodiments, the gene is cloned into an expression vector, such as pET30B to add an N-terminal hexahistidine tag and/or protease cleavage site.

In some embodiments, the polypeptides of the invention may be in substantially purified form. They may be in substantially isolated form, in which case they will generally comprise (for example about or at least) 90%, such as (for example about or at least) 95, 97 or 99% of the polypeptide in the preparation.

The five polypeptides include SEQ ID NOS: 1, 3, 5, 7 and 9.

The subject is not necessarily limiting. In some embodiments, the subject has been infected with *Mycobacterium tuberculosis.* In some embodiments, the subject can have active tuberculosis, latent tuberculosis, or be tuberculosis-negative. A positive result also includes the possibility of a previous infection that has been completely cleared and no possibility of latent tuberculosis. In some embodiments, the method is performed as a screen for tuberculosis in mass populations in developing countries. In some embodiments, the method could be utilized as a community-based triage or referral test to identify people suspected of having tuberculosis. A triage test can be a test that can be used by first-contact providers in the community (for example, community health workers or informal providers) to test persons with any symptoms or risk factors suggestive of tuberculosis who are seeking care (that is, it would not be used for active case-finding) to rule out TB (identify those who are triage-test negative) and direct individuals who require further evaluation (those who are triage-test positive) to a confirmatory test. The triage test can have higher accuracy than symptom screening. Symptom-only screening has very low specificity.

The subject is generally a human but may be an animal, typically one which can be naturally or artificially infected by a mycobacterium. The subject can be a mammal, such as a primate, cow, sheep, pig, badger or rodent, e.g. a mouse or rat. In some embodiments, the subject typically has an active or latent mycobacterial infection, or has had such an infection recently. The subject may test positive or negative in a Mantoux test. In some embodiments, the subject may be at risk of a mycobacterial infection, typically for socioeconomic reasons or may have a genetic or acquired predisposition to mycobacterial infection. In some embodiments, the subject is a healthy contact who has been exposed to a mycobacterium. In some embodiments, the exposure is to pulmonary tuberculosis, such as 'open' pulmonary tuberculosis which is sputum a. f. b. (acid-fast bacillus) smear positive. In some embodiments, the method may be used to trace the healthy contacts of individuals with such tuberculosis infections. The method may also be used to carry out population surveys to measure the number of individuals in a population who have a mycobacterial infection or are healthy contacts.

The method can help distinguish between a subject that has "active" tuberculosis and a subject that has "latent" tuberculosis. Active tuberculosis can be screened wherein the sample from the subject is contacted with SEQ ID NO:9 or an antigenic fragment or variant thereof; SEQ ID NO: 11 or an antigenic fragment or variant thereof; SEQ ID NO: 13 or an antigenic fragment or variant thereof; and SEQ ID NO:15 or an antigenic fragment or variant thereof; followed by detecting formation of antibody-peptide complexes comprising said isolated polypeptides or antigenic fragments or variants thereof; wherein formation of said complexes is indicative of the presence of the antibodies to epitopes of *Mycobacterium tuberculosis* antigens in said sample, wherein the sample is from a subject that has "active" tuberculosis. These B-cell antigens (SEQ ID NO: 9, 11, 13 and 15) induce antibody immune responses that seem to be confined to various categories of "active" TB (smear positive, smear negative, smear negative/culture negative/chest X-ray positive with improvement with treatment) while exhibit little antibody responses (5-10%) in patients with LTBI (smear negative/culture negative/QuantiFERON-TB positive category).

In some embodiments, at least one of the polypeptides, antigenic fragments or variants thereof comprises a non-native sequence, such as an affinity tag to facilitate purification of the polypeptide. In some embodiments, the affinity tag can include 6x-His, HA, GST, or FLAG. In some embodiments, all of the polypeptides, antigenic fragments or variants thereof comprise non-native sequences, such as an affinity tag. At least two of the polypeptides may be conjugated to each other, or part of a fusion protein.

In some embodiments, the accuracy/sensitivity of detecting antibody-peptide complexes in the sample from a subject infected with *Mycobacterium tuberculosis* is at least 70%, at least 80%, at least 90%, or at least 95%.

In some embodiments, the polypeptides are attached to or immobilized upon a solid support. In some embodiments, the solid support is a bead (e.g., a colloidal particle, nanoparticle, latex bead, etc.), a flow path in a lateral flow immunoassay device (e.g., a porous membrane), a flow path in an analytical rotor, or a tube or a well (e.g., in a plate suitable for an ELISA assay). In some embodiments, the solid support comprises metal, glass, a cellulose-based material (e.g., nitrocellulose), or a polymer (e.g., polystyrene, polyethylene, polypropylene, polyester, nylon, polysulfone, etc.). In some embodiments, the peptide or mixture of different peptides is attached to a dendrimer and/or incorporated into a MAPS system.

In some embodiments, the detecting step comprises performing an ELISA assay. In other embodiments, the detecting step comprises performing a lateral flow immunoassay. In other embodiments, the detecting step comprises performing an agglutination assay. In other embodiments, the detecting step comprises spinning the sample in an analytical rotor. In other embodiments, the detecting step comprises analyzing the sample using a Western blot, a slot blot, or a dot blot. In still other embodiments, the detecting step comprises analyzing the sample with an electrochemical sensor, an optical sensor, or an opto-electronic sensor. In some embodiments, the two or more isolated polypeptides or antigenic fragments or variants thereof is conjugated to a ligand. In some embodiments, the two or more isolated polypeptides or antigenic fragments or variants thereof are biotinylated. In some embodiments, the two or more isolated polypeptides or antigenic fragments or variants thereof are conjugated to streptavidin.

Suitable immunoassay methods typically include: receiving or obtaining (e.g., from a patient) a sample of body fluid or tissue likely to contain antibodies; contacting (e.g., incubating or reacting) a sample to be assayed with a peptide of the invention, under conditions effective for the formation of a specific peptide-antibody complex (e.g., for specific binding of the peptide to the antibody); and assaying the contacted (reacted) sample for the presence of an antibody-peptide reaction (e.g., determining the amount of an antibody-peptide complex). The presence of an elevated amount of the antibody-peptide complex indicates that the subject was exposed to and infected with an infectious *Mycobacterium tuberculosis.* A peptide, including a modified form thereof, which "binds specifically" to (e.g., "is specific for" or binds "preferentially" to) an antibody against a *Mycobacterium tuberculosis* antigen interacts with the antibody, or forms or undergoes a physical association with it, in an amount and for a sufficient time to allow detection of the antibody. By "specifically" or "preferentially," it is meant that the peptide has a higher affinity (e.g., a higher degree of selectivity) for such an antibody than for other antibodies in a sample. For example, the peptide can have an affinity for the antibody of at least about 1.5-fold, 2-fold, 2.5-fold, 3-fold, or higher than for other antibodies in the sample. Such affinity or degree of specificity can be determined by a variety of routine procedures, including, e.g., competitive binding studies. In an ELISA assay, a positive response is defined as a value 2 or 3 standard deviations greater than the mean value of a group of healthy controls.

Conditions for reacting peptides and antibodies so that they react specifically are well-known to those of skill in the art. See, e.g., Current Protocols in Immunology (Coligan et al., editors, John Wiley & Sons, Inc).

The methods comprise receiving or obtaining a sample of body fluid or tissue that might contain antibodies from a subject. The antibodies can be, e.g., of IgG, IgE, IgD, IgM, or IgA type. Once the peptide antigen and sample antibody are permitted to react in a suitable medium, an assay is performed to determine the presence or absence of an antibody-peptide reaction. Among the many types of suitable assays, which will be evident to a skilled worker, are immunoprecipitation and agglutination assays.

In some embodiments of the invention, the assay comprises: immobilizing the antibody(s) in the sample, e.g., directly or indirectly via binding to peptides of the invention; adding a peptide of the invention; and detecting the degree of antibody bound to the peptide, e.g., by the peptide being labeled or by adding a labeled substance, such as a labeled binding partner (e.g., streptavidin-colloidal gold complex) or a labeled antibody which specifically recognizes the peptide. In other embodiments, the assay comprises: immobilizing a peptide of the invention; adding the sample containing antibodies; and detecting the amount of antibody bound to the peptide, e.g., by adding another peptide of the invention conjugated, directly or indirectly, to a label (e.g., colloidal gold complex, fluorescent label, enzyme (e.g., horseradish peroxidase or alkaline phosphatase)) or by adding a labeled substance, such as a binding partner or a labeled antibody which specifically recognizes the sample antibodies (e.g., anti-human IgG antibodies, anti-human IgM antibodies, etc.) or combinations thereof. In some embodiments, the assay comprises: immobilizing a peptide of the invention; adding the sample containing antibodies; and detecting the amount of antibody bound to the peptide, e.g., by adding a first binding partner which specifically recognizes the sample antibodies (e.g., anti-human IgG antibodies, anti-human IgM antibodies, etc.), and further adding a second binding partner (e.g., protein A, protein G, protein L, etc.), wherein the second binding partner is labeled and recognizes said first binding partner. In still other embodiments, the assay comprises: reacting the peptide and the sample containing antibodies without any of the reactants being immobilized, and then detecting the amount of complexes of antibody and peptide, e.g., by the peptide being labeled or by adding a labeled substance, such as a labeled binding partner (e.g., streptavidin-colloidal gold complex) or a labeled antibody which specifically recognizes the peptide.

Immobilization of a peptide of the invention can be either covalent or non-covalent, and the non-covalent immobilization can be non-specific (e.g., non-specific binding to a polystyrene surface in, e.g., a microtiter well). Specific or semi-specific binding to a solid or semi-solid carrier, support or surface, can be achieved by the peptide having, associated with it, a moiety which enables its covalent or non-covalent binding to the solid or semi-solid carrier, support or surface. For example, the moiety can have affinity to a component attached to the carrier, support or surface. In this case, the moiety may be, e.g., a biotin or biotinyl group or an analogue thereof bound to an amino acid group of the peptide, such as 6-aminohexanoic acid, and the component is then avidin, streptavidin, neutravidin, or an analogue thereof. An alternative is a situation in which the moiety has the amino acid sequence His-His-His-His-His-His and the carrier comprises a Nitrilo triacetic Acid (NTA) derivative charged with Ni⁺⁺ or Co⁺⁺ ions. In some embodiments, the moiety is a fusion partner.

Suitable carriers, supports, and surfaces include, but are not limited to, beads (e.g., magnetic beads, colloidal particles or nanoparticles, such as colloidal gold, or nanoparticles comprising silica, latex, polystyrene, polycarbonate, or PDVF), latex of co-polymers such as styrene-divinyl benzene, hydroxylated styrene-divinyl benzene, polystyrene, carboxylated polystyrene, beads of carbon black, non-activated or polystyrene or polyvinyl chloride activated glass, epoxy-activated porous magnetic glass, gelatin or polysaccharide particles or other protein particles, red blood cells, mono- or polyclonal antibodies or Fab fragments of such antibodies.

A particularly useful assay format is a lateral flow immunoassay format. Antibodies to human or animal immunoglobulins can be labeled with a signal generator or reporter (e.g., colloidal gold) that is dried and placed on a glass fiber pad (sample application pad or conjugate pad). The diagnostic peptide is immobilized on membrane, such as nitrocellulose or a PVDF (polyvinylidene fluoride) membrane (e.g., an Immobilon membrane). When a solution of sample (blood, serum, etc.) is applied to the sample application pad (or flows through the conjugate pad), it dissolves the labeled reporter, which then binds to all antibodies in the sample. The resulting complexes are then transported into the next membrane (PVDF or nitrocellulose containing the diagnostic peptide) by capillary action. If antibodies against the diagnostic peptide are present, they bind to the diagnostic peptide striped on the membrane, thereby generating a signal (e.g., a band that can be seen or visualized). An additional antibody specific to the labeled antibody or a second labeled antibody can be used to produce a control signal.

An alternative format for the lateral flow immunoassay comprises the peptides or compositions of the invention being conjugated to a ligand (e.g., biotin) and complexed with labeled ligand receptor (e.g., streptavidin-colloidal gold). The labeled peptide complexes can be placed on the sample application pad or conjugate pad. Anti-human IgG/IgM or anti-animal (e.g., dog, mouse, deer) IgG/IgM antibodies or other peptides of the invention are immobilized on a membrane, such as nitrocellulose of PVDF, at a test site (e.g., a test line).

When sample is added to the sample application pad, antibodies in the sample react with the labeled peptide complexes such that antibodies that bind to peptides of the invention become indirectly labeled. The antibodies in the sample are then transported into the membrane (PVDF or nitrocellulose containing the diagnostic peptides) by capillary action and bind to the immobilized anti-human IgG/IgM/IgA or anti-animal IgG/IgM/IgA antibodies (or protein A, protein G, protein L, or combinations thereof) or immobilized peptides of the invention. If any of the sample antibodies are bound to the labeled peptides of the invention, the label associated with the peptides can be seen or visualized at the test site.

Alternatively, when sample is added to the sample application pad, antibodies in the sample react with the labeled anti-human IgG/IgM/IgA or anti-animal IgG/IgM/IgA antibodies (or protein A, protein G, protein L, or combinations thereof) such that antibodies that bind to peptides of the invention become indirectly labeled. The labeled antibodies in the sample are then transported into the membrane (PVDF or nitrocellulose containing the diagnostic peptides) by capillary action and bind to the immobilized peptides of the invention. If any of the sample antibodies are bound to the immobilized peptides of the invention, the label associated with the peptides can be seen or visualized at the test site.

In the embodiments utilizing the lateral flow immunoassay format described above, the lateral flow device may comprise two ports: a sample port, which is positioned between a conjugate pad (containing a labeled analyte-binding partner) and a test site or line (containing an immobilized analyte-binding partner) and a chase port, which is positioned downstream (e.g. toward the end of the device away from test site) of the conjugate pad. In such devices comprising two ports, sample is deposited downstream of the test site via the sample port and fluid flow through the conjugate pad is initiated by depositing solution (e.g. diluent, buffer, or the like) via the chase port. The "chase" solution dissolves the labeled reagents in the conjugate pad and flows through to interact with the sample and then the immobilized reagents at the test site. Alternatively, sample is deposited downstream of the test site via the sample port and migrates toward the test site(s), resulting in the capture of the analyte(s) by the immobilized agents at the test site(s). Subsequently, fluid flow through the conjugate pad is initiated by depositing solution (e.g. diluent, buffer, or the like) via the chase port. The "chase" solution dissolves the labeled reagents in the conjugate pad and flows through to interact with the captured analyte(s) at the test site(s).

In other embodiments utilizing the lateral flow immunoassay format described above, the lateral flow device may comprise one port: a sample/chase buffer port, which is positioned below a conjugate pad (containing a labeled analyte-binding partner) and the test site(s) or line(s) containing immobilized analyte-binding partner(s). In such devices, sample is deposited downstream of the test site via the sample/buffer port and fluid flow through the conjugate pad is initiated by depositing solution (e.g. diluent, buffer, or the like) via the sample/chase buffer port. The "chase" solution dissolves the labeled reagents in the conjugate pad and flows through to interact with the sample and then the immobilized reagents at the test site(s).

Another assay for the screening of blood products or other physiological or biological fluids is an enzyme linked immunosorbent assay, *i*.*e*., an ELISA. Typically, in an ELISA, isolated peptides or compositions of the invention are adsorbed to the surface of a microtiter well directly or through a capture matrix (e.g., an antibody). Residual, non-specific protein-binding sites on the surface are then blocked with an appropriate agent, such as bovine serum albumin (BSA), heat-inactivated normal goat serum (NGS), or BLOTTO (a buffered solution of nonfat dry milk which also contains a preservative, salts, and an antifoaming agent). The well is then incubated with a biological sample suspected of containing specific antibody. The sample can be applied neat, or more often it can be diluted, usually in a buffered solution which contains a small amount (0.1-5.0% by weight) of protein, such as BSA, NGS, or BLOTTO. After incubating for a sufficient length of time to allow specific binding to occur, the well is washed to remove unbound protein and then incubated with an optimal concentration of an appropriate anti-immunoglobulin antibody (e.g., for human subjects, an anti-human immunoglobulin from another animal, such as dog, mouse, cow, etc.) or another peptide of the invention that is conjugated to an enzyme or other label by standard procedures and is dissolved in blocking buffer. The label can be chosen from a variety of enzymes, including horseradish peroxidase (HRP), betagalactosidase, alkaline phosphatase, glucose oxidase, etc. Sufficient time is allowed for specific binding to occur again, then the well is washed again to remove unbound conjugate, and a suitable substrate for the enzyme is added. Color is allowed to develop and the optical density of the contents of the well is determined visually or instrumentally (measured at an appropriate wave length). The cutoff OD value may be defined as the mean OD+3 standard deviations (SDs) of at least 50 serum samples collected from individuals from an area where TB is not endemic, or by other such conventional definitions. In the case of a very specific assay, OD+2 SD can be used as a cutoff value.

In one embodiment of an ELISA, a peptide or peptides of the invention is immobilized on a surface, such as a ninety-six-well ELISA plate or equivalent solid phase that is coated with streptavidin or an equivalent biotin-binding compound, such as avidin or nuetravidin, at an optimal concentration in an alkaline coating buffer and incubated at 4 degrees C overnight. After a suitable number of washes with standard washing buffers, an optimal concentration of a biotinylated form of a peptide or composition of the invention, dissolved in a conventional blocking buffer, is applied to each well. A sample is then added, and the assay proceeds as above. Conditions for performing ELISA assays are well-known in the art.

In another embodiment, the methods comprise an agglutination assay. For example, in some embodiments, colloidal particles (e.g., colloidal gold, etc.) or latex beads are conjugated to peptides or compositions of the invention. Subsequently, the biological fluid is incubated with the bead/peptide conjugate, thereby forming a reaction mixture. The reaction mixture is then analyzed to determine the presence of the antibodies. In some embodiments, the agglutination assays comprise the use of a second population of particles, such as colloidal particles (e.g., colloidal gold, etc.) or latex beads, conjugated to (1) antibodies specific to the peptides of compositions of the invention, in the case of a competition assay, or (2) antibodies capable of detecting sample antibodies (e.g., anti-human IgG or IgM or IgA antibodies, etc.), in the case of a sandwich assay. Suitable agglutination methods can comprise centrifugation as a means of assessing the extent of agglutination.

It should be understood by one of skill in the art that any number of protein assay formats, particularly immunoassay formats, may be designed to utilize the isolated peptides of this invention for the detection of antibodies and infection in a subject. This invention is thus not limited by the selection of the particular assay format, and is believed to encompass assay formats that are known to those of skill in the art.

In another embodiment, the invention provides a method for assaying interferon-γ production from a sample comprising T cells from a subject, comprising
i. contacting the sample comprising T cells from the subject with five polypeptides comprising SEQ ID NOS:1, 3, 5, 7 and 9; and
ii. detecting the presence of interferon-γ produced by the T cells.

Disclosed but not claimed is a method for detecting interferon-γ producing T cells from a sample from a subject, comprising
i. contacting the sample comprising T cells from the subject with two or more isolated polypeptides or antigenic fragments or variants thereof, wherein the polypeptides comprise polypeptides selected from the group consisting of SEQ ID NOS: 1, 3, 5, 7, 9, 11, 13, and 15; and
ii. detecting the presence of interferon-γ producing T cells.

The T cells which recognize the peptide in the method are generally T cells which have been pre-sensitized *in vivo* to antigen from a mycobacterium. In some embodiments, these antigen-experienced T cells are generally present in the peripheral blood of a host which has been exposed to the mycobacterium at a frequency of 1 in 10⁶ to 1 in 10³ peripheral blood mononuclear cells (PBMCs). The T cells may be CD4+ and/or CD8+ T cells.

In the method the T cells can be contacted with the peptides *in vitro* or *in vivo,* and determining whether the T cells recognize the peptide can be done in vitro or in vivo.

Determination of whether the T cells recognize the peptide is generally done by detecting an increased secretion of cytokine, such as interferon-γ, in the presence of the peptide. The interferon-γ can typically be detected by allowing it to bind to a specific binding agent and then measuring the presence of the specific binding agent/ interferon-γ complex. The specific binding agent is typically an antibody, such as polyclonal or monoclonal antibodies. Antibodies to interferon-γ are commercially available, or can be made using standard techniques.

In some embodiments, the specific binding agent for interferon-γ is immobilized on a solid support. After the substance is allowed to bind the solid support it can optionally be washed to remove material which is not specifically bound to the agent. The agent/substance complex can be detected by using a second binding agent which will bind the complex. In some embodiments, the second agent binds the substance at a site which is different from the site which binds the first agent. The second agent can be an antibody that is labeled directly or indirectly by a detectable label.

In some embodiments, the second agent can be detected by a third agent which is typically labeled directly or indirectly by a detectable label. For example, the second agent may comprise a biotin moiety, allowing detection by a third agent which comprises a streptavidin moiety and typically alkaline phosphatase as a detectable label.

In one embodiment the detection system which is used is the ex-vivo ELISPOT assay described in WO 98/23960. In that assay interferon-γ secreted from the T cell is bound by a first interferon-γ specific antibody which is immobilized on a solid support. The bound interferon-γ is then detected using a second interferon-γ specific antibody which is labeled with a detectable label. Such a labeled antibody can be obtained from MABTECH (Stockholm, Sweden). Other detectable labels which can be used are discussed below.

In some embodiments, the T cells which are contacted in the method are taken from the host in a blood sample, although other types of samples which contain T cells can be used. The sample may be added directly to the assay or may be processed first. Typically, the processing may comprise diluting of the sample, for example with water or buffer. Typically, the sample is diluted from 1.5 to 100 fold, for example 2 to 50 or 5 to 10-fold.

The processing may comprise separation of components of the sample. Typically, mononuclear cells (MCs) are separated from the samples. The MCs will comprise the T cells and APCs. In some embodiments of the method the APCs present in the separated MCs can present the peptide to the T cells. In another embodiment only T cells, such as only CD4 or only CD8 T cells, can be purified from the sample. PBMCs, MCs and T cells can be separated from the sample using techniques known in the art, such as those described in Lalvani et al (1997) J. Exp. Med. 186, p 859-865.

In some embodiments, the T cells used in the assay are in the form of unprocessed or diluted whole blood samples, or are freshly isolated T cells (such as in the form of freshly isolated MCs or PBMCs) which are used directly *ex vivo,* i.e. they are not cultured before being used in the method. However, the T cells can be cultured before use, for example in the presence of one or more of the peptides, and generally also exogenous growth promoting cytokines During culturing the peptides are typically present on the surface of APCs, such as the APC used in the method. Pre-culturing of the T cells can lead to an increase in the sensitivity of the method.

The APC which can be present in the method can be from the same host as the T cell or from a different host. The APC can be a naturally occurring APC or an artificial APC. The APC is a cell which is capable of presenting the peptide to a T cell. It is typically a B cell, dendritic cell or macrophage. It is typically separated from the same sample as the T cell and is typically co-purified with the T cell. Thus, the APC may be present in MCs or PBMCs. The APC is typically a freshly isolated *ex vivo* cell or a cultured cell. It may be in the form of a cell line, such as a short term or immortalized cell line. The APC may express empty MHC class II molecules on its surface.

In some embodiments, the T cells derived from the sample can be placed into an assay with all the peptides (*i.e.* a pool of the peptides) which it is intended to test (the relevant panel) or the T cells can be divided and placed into separate assays each of which contain one or more of the peptides.

In one embodiment peptide per se is added directly to an assay comprising T cells and APCs. As discussed above the T cells and APCs in such an assay could be in the form of MCs. When peptides which can be recognized by the T cell without the need for presentation by APCs are used then APCs are not required. Analogues which mimic the original peptide bound to a MHC molecule are an example of such a peptide.

In one embodiment the peptide is provided to the APC in the absence of the T cell. The APC is then provided to the T cell, typically after being allowed to present the peptide on its surface. The peptide may have been taken up inside the APC and presented, or simply be taken up onto the surface without entering inside the APC.

The duration for which the peptide is contacted with the T cells will vary depending on the method used for determining recognition of the peptide. Typically, 10⁵ to 10⁷, preferably 5×10⁵ to 10⁶ PBMCs are added to each assay. In the case where peptide is added directly to the assay its concentration is from 10⁻¹ to 10³ µg/ml, preferably 0.5 to 50 µg/ml or 1 to 10 µg/ml.

Typically, the length of time for which the T cells are incubated with the peptide is from 4 to 24 hours, preferably 6 to 16 hours.

### Compositions and Devices

In another embodiment, the invention provides devices. In some embodiments, the devices are useful for performing an immunoassay. For example, in some embodiments, the device is a lateral flow immunoassay device. In other embodiments, the device is an analytical rotor. In other embodiments, the device is a dot blot, slot blot, or Western blot. In other embodiments, the device is a tube or a well, e.g., in a plate suitable for an ELISA assay. In still other embodiments, the device is an electrochemical sensor, an optical sensor, or an opto-electronic sensor.

The device comprises polypeptides, antigenic fragments or variants of *Mycobacterium tuberculosis* antigens as defined by the claims. In some embodiments, the peptides are attached to or immobilized upon the device. In some embodiments, peptides of the invention are attached to or immobilized on a substrate, such as a solid or semi-solid support. The attachment can be covalent or non-covalent, and can be facilitated by a moiety associated with the peptide that enables covalent or non-covalent binding, such as a moiety that has a high affinity to a component attached to the carrier, support or surface. For example, the peptide can be associated with a ligand, such as biotin, and the component associated with the surface can be a corresponding ligand receptor, such as avidin. In some embodiments, the peptide can be associated with a fusion partner, e.g., bovine serum albumin (BSA), which facilitates with the attachment of the peptide to a substrate. The peptide can be attached to or immobilized on the substrate either prior to or after the addition of a sample containing antibody during an immunoassay.

In some embodiments, the substrate is a bead, such as a colloidal particle (e.g., a colloidal nanoparticle made from gold, silver, platinum, copper, metal composites, other soft metals, core-shell structure particles, or hollow gold nanospheres) or other type of particle (e.g., a magnetic bead or a particle or nanoparticle comprising silica, latex, polystyrene, polycarbonate, polyacrylate, or PVDF). Such particles can comprise a label (e.g., a colorimetric, chemiluminescent, or fluorescent label) and can be useful for visualizing the location of the peptides during immunoassays. In some embodiments, a terminal cysteine of a peptide of the invention is used to bind the peptide directly to the nanoparticles made from gold, silver, platinum, copper, metal composites, other soft metals, etc.

In some embodiments, the substrate is a dot blot or a flow path in a lateral flow immunoassay device. For example, the peptides can be attached or immobilized on a porous membrane, such as a PVDF membrane (e.g., an Immobilon.TM. membrane), a nitrocellulose membrane, polyethylene membrane, nylon membrane, or a similar type of membrane.

In some embodiments, the substrate is a flow path in an analytical rotor. In other embodiments, the substrate is a tube or a well, such as a well in a plate (e.g., a microtiter plate) suitable for use in an ELISA assay. Such substrates can comprise glass, cellulose-based materials, thermoplastic polymers, such as polyethylene, polypropylene, or polyester, sintered structures composed of particulate materials (e.g., glass or various thermoplastic polymers), or cast membrane film composed of nitrocellulose, nylon, polysulfone, or the like. A substrate can be sintered, fine particles of polyethylene, commonly known as porous polyethylene, for example, 0.2-15 micron porous polyethylene from Chromex Corporation (Albuquerque, N. Mex.). All of these substrate materials can be used in suitable shapes, such as films, sheets, or plates, or they may be coated onto or bonded or laminated to appropriate inert carriers, such as paper, glass, plastic films, or fabrics. Suitable methods for immobilizing peptides on solid phases include ionic, hydrophobic, covalent interactions and the like.

Disclosed but not claimed is a composition comprising mixtures of at least two isolated polypeptides, fragments and/or variants thereof capable of binding to antibodies that recognize *Mycobacterium tuberculosis* antigens. The polypeptides are selected from the group consisting of SEQ ID NOS:1, 3, 5, 7, 9, 11, 13, 15 and combinations thereof. The polypeptides comprise an affinity tag to facilitate purification of the polypeptide. The affinity tag is a 6x-Histidine tag, which can be present on the N- and/or C-terminus of the protein. The mixtures of polypeptides can be useful, *e.g.,* in diagnostic assays to detect antibodies that recognize one or more epitopes on SEQ ID NOS: 1, 3, 5, 7, 9, 11, 13, and 15. The composition comprises a mixture of 2, 3, 4, 5, 6, 7, 8, 9, 10, 15, 20, 25, 30, 40, 50, 60, 70, 80, 90, 100, 150, 200, 250, 300, 400, 500, or more polypeptides, antigenic fragments and variants thereof as described herein. IThe peptides are modified (e.g., by association with one or more further moieties), as described herein.

Disclosed but not claimed are nucleic acids comprising sequences encoding the polypeptides, antigenic fragments and variants thereof of the invention. Nucleic acids contain less than an entire microbial genome and can be single- or double-stranded. A nucleic acid can be RNA, DNA, cDNA, genomic DNA, chemically synthesized RNA or DNA or combinations thereof. The nucleic acids can be purified free of other components, such as proteins, lipids and other polynucleotides. For example, the nucleic acids can be 50%, 75%, 90%, 95%, 96%, 97%, 98%, 99%, or 100% purified. The nucleic acids of the invention encode the peptides described herein. In some embodiments, the nucleic acids encode a peptide having the sequence of SEQ ID NO: 1, 3, 5, 7, 9, 11, 13 and 15 or combinations thereof. Nucleic acids can comprise other nucleotide sequences, such as sequences coding for linkers, signal sequences, TMR stop transfer sequences, transmembrane domains, or ligands useful in protein purification such as glutathione-S-transferase, histidine tag, and staphylococcal protein A.

Nucleic acids can be isolated. An "isolated" nucleic acid is one that is not immediately contiguous with one or both of the 5' and 3' flanking genomic sequences that it is naturally associated with. An isolated nucleic acid can be, e.g., a recombinant DNA molecule of any length, provided that the nucleic acid sequence naturally found immediately flanking the recombinant DNA molecule in a naturally-occurring genome is removed or absent. Isolated nucleic acids can also include non-naturally occurring nucleic acid molecules. Nucleic acids of the invention can also comprise fragments that encode immunogenic peptides. Nucleic acids of the invention can encode full-length polypeptides, peptide fragments, and variant or fusion peptides.

Nucleic acids can be isolated, at least in part, from nucleic acid sequences present in, for example, a biological sample, such as blood, serum, saliva, or tissue from an infected individual. Nucleic acids can also be synthesized in the laboratory, for example, using an automatic synthesizer. An amplification method such as PCR can be used to amplify nucleic acids, at least in part, from either genomic DNA or cDNA encoding the polypeptides.

Nucleic acids can comprise coding sequences for naturally occurring polypeptides or can encode altered sequences that do not occur in nature.

### Vectors, Host Cells and Recombinant Expression

Disclosed but not claimed are vectors that comprise a polypeptides, antigenic fragments or variants thereof from *Mycobacterium tuberculosis,* host cells which are genetically engineered to express the polypeptides, antigenic fragments or variants thereof and the production of polypeptides, antigenic fragments or variants thereof of the invention by recombinant techniques. Cell-free translation systems can also be employed to produce such proteins using RNAs derived from the DNA constructs of the invention.

When a polynucleotide encoding a polypeptide, antigenic fragment or variant thereof from *Mycobacterium tuberculosis* is used for the recombinant production of a polypeptide, the polynucleotide may include the coding sequence for the full-length polypeptide or an antigenic fragment or variant thereof, by itself; the coding sequence for the full-length polypeptide, fragment or variant thereof in reading frame with other coding sequences, such as those encoding a leader or secretory sequence, a pre-, or pro or preproprotein sequence, or other fusion peptide portions. For example, an affinity tag that facilitates purification of the fused polypeptide can be encoded. In certain embodiments of this aspect of the invention, the marker sequence is a hexa-histidine peptide, for example, as provided in the pQE vector (Qiagen, Inc.) and described in Gentz et al., Proc Natl Acad Sci USA 86:821-824 (1989), or it may be the HA tag, which corresponds to an epitope derived from the influenza hemagglutinin protein (Wilson, I., et al., Cell 37:767, 1984). The polynucleotide may also contain non-coding 5' and 3' sequences, such as transcribed, non-translated sequences, ribosome binding sites and sequences that stabilize mRNA.

Representative examples of appropriate hosts include bacterial cells, such as streptococci, staphylococci, *E. coli, Streptomyces, Bacillus subtilis,* and fungal cells, such as yeast cells and *Aspergillus* cells. In some embodiments, gram negative bacteria are the host cells. A great variety of expression systems can be used, including DNA or RNA vectors. In other embodiments, the invention provides an isolated nucleic acid molecule comprising a type IV pilin operably linked to a heterologous promoter. In some embodiments, the invention further provides an isolated *Mycobacterium tuberculosis* nucleic acid molecule operably linked to a heterologous promoter, wherein said isolated nucleic acid molecule is capable of expressing a polypeptide when used to transform an appropriate host cell.

Disclosed but not claimed is an isolated nucleic acid molecule encoding an antigenic fragment or variant of *Mycobacterium tuberculosis* polypeptide linked to an affinity tag sequence and/or enzymatic cleavage sequence to facilitate purification. In some embodiments, the affinity tag is a 6x-Histidine tag. The cleavage sequence is recognized by enterokinase. The nucleic acid molecules are optimized to increase expression in *E*. *coli* without altering the amino acid sequence using preferred codons in *E. coli.* Disclosed are purified polypeptides, variants and antigenic fragments of a *Mycobacterium tuberculosis* polypeptide as described herein.

The amino acid sequence to be expressed is reverse translated for codon optimization in *E. coli* using a commercial tool and synthesized along with flanking restriction sites. In some embodiments, the synthetic gene is cloned into an expression vector, such as pET30B to add an N-terminal hexahistidine tag and protease cleavage site.

### EXAMPLES

### Example 1. Mycobacterium tuberculosis regions of difference

Comparative genomic studies have identified several *Mycobacterium tuberculosis-*specific genomic regions of difference (RDs) which are absent in the vaccine strains of *Mycobacterium bovis* BCG and which may be useful for the diagnosis of TB. Detailed information on ORF present in various RD, distributions of RD in various mycobacterial species including BCG strains, *M. tuberculosis* and *M. bovis* isolates are shown in Tables 1-3. These Tables are obtained from O. Parkash et al., Scandinavian Journal of Immunology 2009; 70(4), 345-357. The proteins encoded by these regions are called RD proteins. In recent years, a number of RD antigens have been investigated for their potential in T-cell-based cell-mediated immune response (CMI) assays. Particularly, these RD antigens have attracted attention for their potential in improving serological assays for tuberculosis diagnosis. Our work in this area has identified a number of antigens in various RDs and related *M. tuberculosis* genomic regions for use in serological diagnostics. The best performing antigens for use in serological TB assays that we have uncovered include the Rv0222, Rv3120, Rv1989c, Rv3118, Rv1636, Rv3426, Rv2185c, Rv3354 and others.

**Table 1. Characteristics of regions of differences (RD) present in Mycobacterium tuberculosis.**

| **RD** | **Size of ORF (kb)** | **ORF (number)** | **ORF (range)** | **Gene Segment(s)** | **ORF (bp range)** |
|---|---|---|---|---|---|
| Abbreviation: ORF, open reading frames. | | | | | |
| 1 | 9.5 | 9 | Rv3871-Rv3879c | 160 | 7534-16989 |
| 2 | 5.6 | 11 | Rv1978-Rv1988 | 88-89 | 14211-8598 |
| 3 | 9.3 | 14 | Rv1573-Rv1586c | 70 | 7677-16923 |
| 4 | 1.9 | 3 | Rv0221-Rv0223c | 12 | 17432-19335 |
| 5 | 2.8 | 5 | Rv3117-Rv3121 | 135 | 27437-30212 |
| 6 | 12.8 | 11 | Rv1506c-Rv1516c | 65 | 23614-36437 |
| 7 | ~9.0 | 8 | Rv2346c-Rv2353c | 103 | 17622-26584 |
| 8 | 3.4 | 4 | Rv0309-Rv0312 | 16 | 17018-20446 |
| 9 | 18.3 | 7 | Rv3617-Rv3623 | 153-154 | 21131-2832 |
| 10 | 3.0 | 3 | Rv1255c-Rv1257c | 55 | 3689-6696 |
| 11 | 28.8 | 5 | Rv3425-Rv3429 | 145-146 | 30303-1475 |
| 12 | 2.0 | 4 | Rv2072c-Rv2075c | 93 | 9301-11331 |
| 13 | ~11.0 | 16 | Rv2645-Rv2660c | 118 | 12475-23455 |
| 14 | ~9.1 | 8 | Rv1766-Rv1773c | 79 | 30573-39642 |
| 15 | 12.7 | 15 | Rv1963c-Rv1977 | 88 | 1153-13873 |
| 16 | 7.6 | 6 | Rv3400-Rv3405c | 145 | 5012-12621 |

**Table 2. Distributions of RD regions in various mycobacteria.**

| **Mycobacteria** | **Regions of differences (RD)** | | | | | | | | | | | | | | | |
|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** |
| Abbreviation: NR - no report. | | | | | | | | | | | | | | | | |
| *Mycobacterium tuberculosis* H37Rv | + | + | + | + | + | + | + | + | + | + | + | + | + | NR + | | NR |
| *M. tuberculosis Erdman* | + | + | + | NR | NR | NR | NR | NR | NR | NR | NR | NR | NR | NR | NR | NR |
| *M. africanum* | NR | NR | NR | + | + | + | + | + | - | + | NR | NR | NR | NR | NR | NR |
| *M. bovis* | + | + | + | - | - | - | - | - | - | - | NR | NR | + | NR | NR | NR |
| *M. microti* OV254 | NR | NR | NR | + | - | - | - | - | - | - | NR | NR | NR | NR | NR | NR |
| *M. tuberculosis* CSU93 | NR | NR | NR | + | + | - | + | + | + | + | NR | NR | NR | NR | NR | NR |
| *M. smegmatis* | - | - | - | NR | NR | NR | NR | NR | NR | NR | NR | NR | NR | NR | NR | NR |
| *M. avium* | - | - | - | NR | NR | NR | NR | NR | NR | NR | NR | NR | NR | NR | NR | NR |

**Table 3. Distributions of RD regions in various BCG strains.**

| **BCG Strains** | **Regions of Differences (RD)** | | | | | | | | | | | | | | | |
|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** |
| Denote: +, present; -, absent; NR, no report; BCG, Bacillus Calmette Guerin. | | | | | | | | | | | | | | | | |
| Source: Based on references 19, 20. | | | | | | | | | | | | | | | | |
| BCG-Danish | - | - | - | - | - | - | - | + | - | - | - | - | - | + | - | + |
| BCG-Prague | - | - | - | - | - | - | - | + | - | - | - | - | - | + | - | + |
| BCG-Glaxo | - | - | - | - | - | - | - | + | - | - | - | - | - | + | - | + |
| BCG-Frappier | - | - | - | - | - | - | - | - | - | - | - | - | - | + | - | + |
| BCG-Connauht | - | - | - | - | - | - | - | - | - | - | - | - | - | + | - | + |
| BCG-Phipps | - | - | - | - | - | - | - | + | - | - | - | - | - | + | - | + |
| BCG-Tice | - | - | - | - | - | - | - | + | - | - | - | - | - | + | - | + |
| BCG-Pasteur | - | - | - | - | - | - | - | + | - | - | - | - | - | - | - | + |
| BCG-Moreau | - | + | - | - | - | - | - | + | - | - | - | - | - | + | - | - |
| BCG-Birkhaug | - | + | - | - | - | - | - | + | - | - | - | - | - | + | - | + |
| BCG-Sweden | - | + | - | - | - | - | - | + | - | - | - | - | - | + | - | + |
| BCG-Japan | - | + | - | - | - | - | - | + | - | - | - | - | - | + | - | + |
| BCG-Russia | - | + | - | - | - | - | - | + | - | - | - | - | - | + | - | + |
| BCG-Brazil | - | + | - | NR | NR | NR | NR | NR | NR | NR | NR | NR | NR | NR | NR | NR |

### Example 2. Obtaining Purified, Recombinant, Full-Length Rv0222, Rv3120, and Rv1989c

The full-length, correctly folded *M. tuberculosis* proteins Rv0222, Rv3120, Rv1989c, Rv3118, Rv1636, Rv3426 can be obtained as recombinant versions expressed in *E. coli* or any other suitable recombinant protein overexpression host organisms. A method to obtain significant quantities of purified aforementioned antigens from *E. coli* cells in the inventors' laboratory is described here. A DNA segment encoding the full-length Rv0222 or Rv3120, or Rv1989c or Rv3118 or Rv1636 or Rv3426 amino acid sequence (Table 4) is cloned into an *E. coli* protein expression of choice. Examples of suitable expression vectors include the pJexpress vector - an *E. coli* expression vector utilizing T5 phage promoter or our proprietary expression vector pMOS containing the pmo1 promoter. The expression vector is subsequently transformed into *E. coli* cells. After the recombinant protein expressing cells have been cultured and collected, the recombinant cells are lysed with an appropriate method. The cell lysate is centrifuged at 12K rpm for 15 min to separate the soluble extract from the particulate fraction. If the recombinant protein is expressed as a soluble protein, the recombinant protein can be purified from the soluble extract using chromatography, for instance, metal affinity chromatography, taking advantage of the 6xHis tag at the C-terminus of the recombinant protein. If the protein is expressed as inclusion bodies, the pellet fraction obtained after centrifugation step, can be solubilized with solution containing 8M urea, refolded by slow dialysis to remove urea, and then purified using metal affinity chromatography, taking advantage of the 6xHis tag at the C-terminus of the recombinant protein.

### Example 3. Embodiments of the TB diagnostics Test - Rapid Lateral Flow Tests Utilizing the Rv0222, Rv3120, and Rv1989c and their combination with other antigens

An embodiment of the invention for the use of the Rv0222, Rv3120, and Rv1989c proteins is the rapid lateral flow chromatographic test to diagnose Tuberculosis. The so-called rapid tests introduced first in the 1980s are inexpensive, disposable, membrane-based lateral flow assays that provide visual evidence of the presence of an analyte in a patient sample. The tests can be formatted either as freestanding dipsticks or as devices enclosed within plastic housings. As little as 10-100 µl of liquid sample is required to perform the test, which can be completed within 5-15 minutes. In clinical tests, the sample may be a small volume of whole blood, serum, plasma, saliva, or urine depending on the type of assay product and marker to be detected. These can be applied directly to the sample application wick for dipstick device or sample well for card device. No instrumentation is required to perform such tests, which can be used in clinics, laboratories, field locations, and the home, often by inexperienced personnel. Optional use of portable table-top or handheld optical density or fluorescence intensity analyzing instruments is also available.

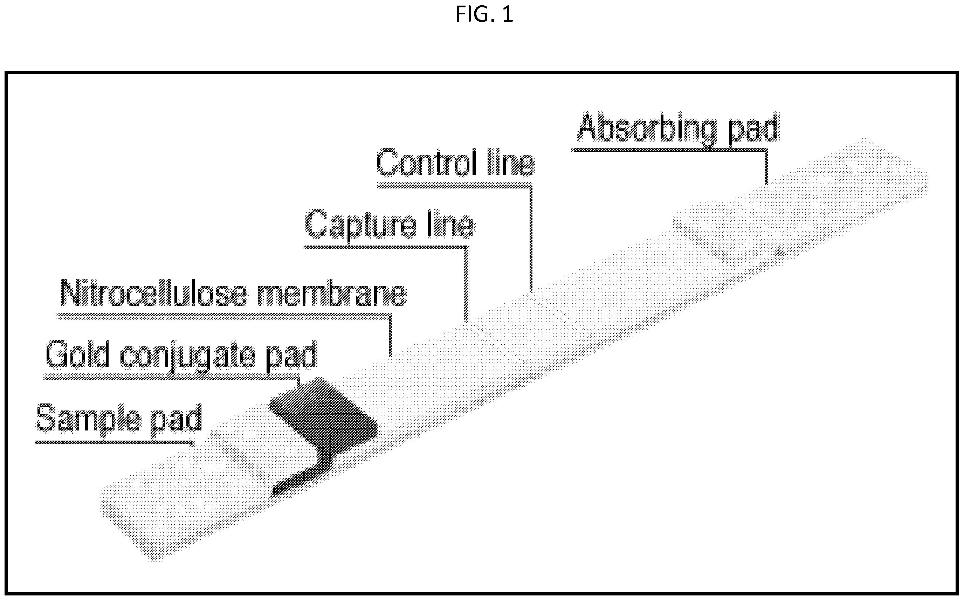

The "basic" (or classical) architecture of the lateral flow rapid test strips is shown in Figure 1. The basic test strips have these components: sample pad, conjugate pad, the nitrocellulose membrane (where the antigen and control materials are printed on), backing card, absorbent wick, and cover tape if desired (Figure 1). The appropriate sample pad material (eg. Cytosep or untreated paper or glass fiber), conjugate pad material (polyester or glass fiber), membrane (nitrocellulose), and absorbent wick (paper) as well as their dimension were optimized to determine the best combination to yield to best flow characteristics and reactivity. An additional mixing pad is also employed in many embodiment of the lateral flow rapid test strip.

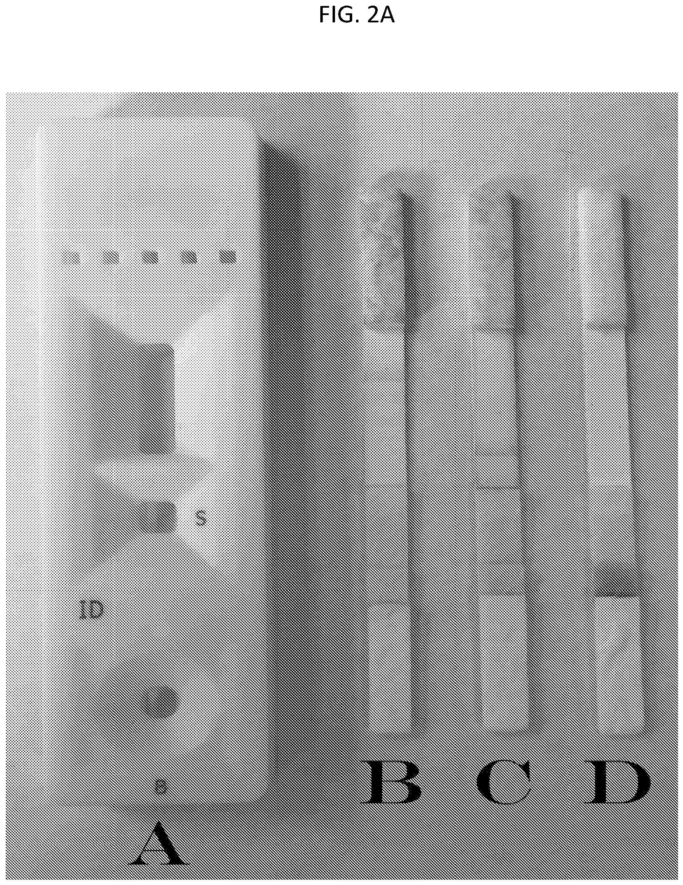

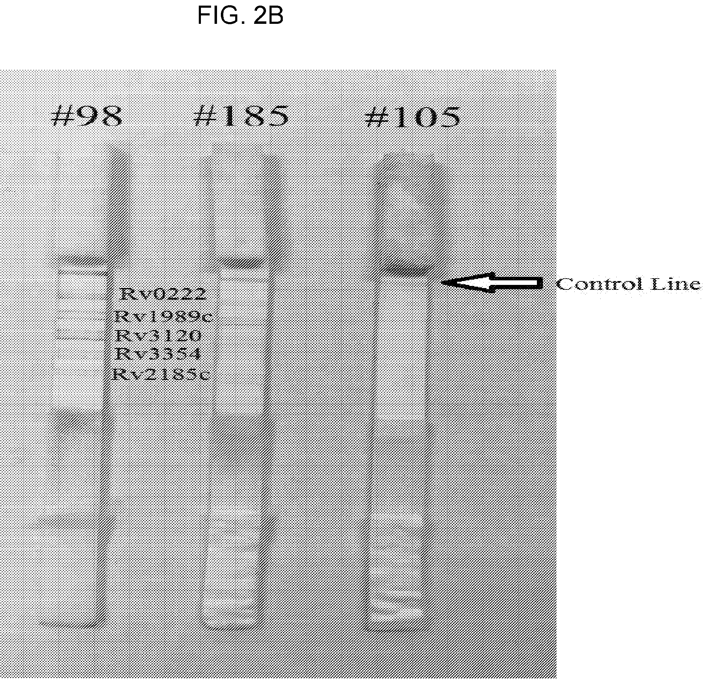

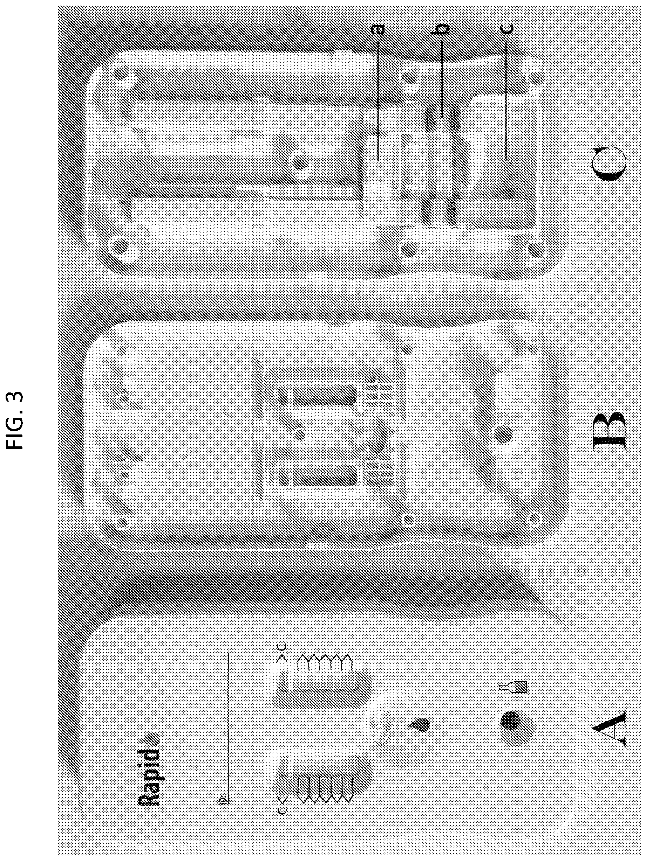

The rapid lateral flow assays are also capable of detecting multiple analytes simultaneously on a single strip. Tests with up to 5 different analytes on the same strip have been developed and marketed. For our TB serodiagnostic tests, the base substrate is a nitrocellulose membrane onto which the capture antigen(s) is (are) immobilized (Figure 1, capture line or test line). For usage convenience, our TB lateral flow test would have three to five test lines with one different capture antigen (Rv0222 or Rv3120 or Rv1989c or Rv3354 or Rv2185c or Rv3118 or Rv1636 or Rv3426 or their various combinations - Figure 2) on each test line. A pad containing dried conjugate is attached to the membrane strip. For this particular embodiment of our Tuberculosis test, this conjugate pad contains gold or fluorescent or magnetic nanoparticles conjugated with goat anti-human anti-IgG or anti-IgM or anti-IgA antibodies. The sample pad can also have an additional function of blood separation. This will allow 3 types of blood samples - whole blood, plasma or serum to be used. When applied to the sample pad, the liquid sample migrates by capillary diffusion through the conjugate pad, rehydrating the conjugates and allowing the interaction of the sample analyte with the conjugates. The complex of conjugates and analytes (*M. tuberculosis* antigen-specific IgG and IgM antibodies) then moves onto the membrane strip and migrates towards the capture proteins, where the complexes become immobilized and produce a distinct signal in the form of a red/purple line if gold conjugates are used. A control line forms regardless of target analyte(s) presence or absence in the specimen indicating the test is complete. In the case that all 6 antigens and/or additional antigens are desired to be present in the TB test, dual-path lateral flow test design (Figure 3) can be utilized, which will allow up 10 antigens to be evaluated in a single assay.

There are many choices for the conjugates to be used in the assays - magnetic nanoparticles, fluorescent nanoparticles in addition to the standard of the industry - gold conjugates. As a result of their greater stability, sensitivity, precision and reproducibility, gold conjugates were introduced into membrane-based rapid tests in the late 1980s and have become the industry mainstay. Gold particles of any accurately defined size can be manufactured reproducibly under the appropriate manufacturing conditions. The visual signal is generated on the test strip by the accumulation of gold particles by the specific immunochemical reaction at the test (capture) or control line. Generally, the larger the gold particles are, the better visualization occurs. However, the trade-off between required visibility and steric hindrance dictates that, for most immunoassay applications, the optimum particle size is 40-60 nm. Larger particles may be preferred when a darker red color is desired or when the lower curvature of the particle surface would improve the molecular interaction between the antibody and antigen.

### Example 4. TB Serodiagnostic Assays

The major issue with current TB serodiagnostics/rapid tests is that these tests are known to be of inferior quality. Mostly TB rapid serodiagnostics are produced out of China and India, but poor quality standards and sloppy practices have resulted in a huge range of accuracy data, rendering them all but essentially useless.

In order to correct for this situation, the first issue in order to develop better TB serodiagnostics would be the selection of better antigens that can be used in the serological assay. Although the accuracy of existing serological rapid assays is poor and these tests have not been clinically useful, efforts towards discovery of novel TB antigens and better strategies for developing efficient serodiagnostics have continued unabatedly in the hope of discovering better-performing markers/antigens. If such a POC, highly sensitive, inexpensive, rapid serodiagnostic test could ever be developed, even with a moderate specificity (80%-90%), the impact could be very significant. These tests could be used as screening tests to triage the patients into stratified groups so that the more expensive but "definitive" testing methods (like culture or the Xpert MTB/RIF) can be focused on the highly suspected cases. Such tests would also be very useful for large-scale active case-finding approaches, where current tests are prohibitively expensive.

In order to come up with better TB antigens, we have screened by IgM/IgG ELISA a collection of smear-positive samples (>200 serum samples from various TB endemic countries) for promising antigen candidates. The antigens screened include approximately 75% of all proteins belonging to the Regions of Difference (RDs) of the *M. tuberculosis* genome and their vicinities, numerous antigens not in the RDs but found in various literature sources to be good antigens, as well as TB antigens known to have been utilized in other TB serodiagnostics/rapid tests. The results from these IgG/IgM screening experiments indicated the following: 1) Very few single antigen that we have investigated (>100 candidates) could provide a positive reading with >40% of the samples (both IgM and IgG). 2) TB antigens known to have been utilized previously in TB serodiagnostics/rapid tests currently in the market exhibited either very poor or poor results (20-30% positive detection rate, combined IgM and IgG). 3) A minimal combination of 3 best performing antigens was needed in order to detect >80% of the samples. Eventually we settled on a best combination of the most immunogenic antigens, Rv0222, Rv3120, Rv1989c, Rv2185c, and Rv3354 (Panel I). These antigens are among the most immunodominant ones that we were able to identify. Our Panel II antigens comprise of the following antigens, Rv3118, Rv1636, Rv3426, and Rv3120, which were discovered in a later screening using samples that we received from the World Health Organization (WHO).

Our TB diagnostics prototypes were evaluated by blind testing using a TB sample collection provided by the WHO. This collection contains samples in the following categories: smear positive/culture positive, smear negative/culture positive, smear negative/culture negative/chest radiology X-ray (CRX) positive (with improvement upon treatment), smear negative/culture negative/Quantiferon positive (latent TB), and non-TB (smear negative/culture negative). These samples were collected from patients from South Africa, Brazil, Peru and Vietnam.

After blind testing following a previously described protocol by the WHO (http://www.who.int/tdr/publications/documents/diagnostic-evaluation-2.pdf), the results obtained with the Panel I TB serodiagnostic prototypes can be summarized as following:
1) In the smear positive/culture positive group, our test detected 95% of these samples (n=60);
2) In the smear negative/culture positive group, our test detected 98% of these samples (n=40);
3) In the smear negative/culture negative/CRX positive group, our test detected 100% of these samples (n=15);
4) In the smear negative/culture negative/quantiferon-TB positive, our test detected 100% of these samples (n=20);
5) In the so-called Non-TB group (smear negative/culture negative), our TB test correctly predicted TB-negative status in 82% of these samples (n=60).

The "non-TB" samples in the WHO collection were evaluated only by smear microscopy and culture methods and therefore probably have included patients with latent TB. A more accurate term to refer to these "non-TB" cases is "non-active" TB. As such, the positive responses in this so-called "non-TB" group are actually LTBI cases. These testing results indicate that our Panel I TB test probably is a point-of-care equivalent of the IGRA QuantiFERON-TB test.

Our Panel 2 TB test prototypes were also evaluated using the WHO sample collection, and the following results were obtained:
1) The detection rates in the smear negative/culture positive, smear negative/culture negative/CRX positive categories are still >90%-95%;
2) The detection rate in the latent TB category (smear negative/culture negative/Quantiferon positive) is low, only 10%;
3) In the smear positive/culture positive category, the TB 2 test detected 93% of these samples while maintaining a specificity of 90% in the non-TB group.

Our TB diagnostics comprising of two Panels which can be combined into a single test or separated into 2 tests as mentioned in the Device Description Section. The test utilizing Panel I antigens is the POC equivalent of the QuantiFeron-TB test, capable of detecting almost all TB cases, including active TB and LTBI. The Panel I TB test should perform very well with HIV/TB co-infection cases since three antigens in this panel (Rv0222, Rv3154, Rv2185c) have been found to yield 85% or better responses in the TB/HIV category where current commercial serodiagnostics have performed poorly.

## Claims

1. A method of detecting antibodies in a sample from a subject, wherein the antibodies bind to epitopes of *Mycobacterium tuberculosis* antigens, comprising
i. contacting the sample with five polypeptides comprising SEQ ID NOS: 1, 3, 5, 7 and 9; and
ii. detecting formation of antibody-peptide complexes comprising said polypeptides;
wherein formation of said complexes is indicative of the presence of the antibodies to epitopes of *Mycobacterium tuberculosis* antigens in said sample.

2. The method of claim 1, wherein the subject has been infected with *Mycobacterium tuberculosis.*

3. The method of any of claims 1-2, wherein the sample is selected from the group consisting of blood, serum, plasma, lymph nodes, skin, saliva, urine, cerebrospinal fluid and milk.

4. A device for assaying for the presence of antibodies in a sample from a subject, wherein the antibodies bind to epitopes of *Mycobacterium tuberculosis* antigens, wherein the device comprises five polypeptides comprising SEQ ID NOS: 1, 3, 5, 7 and 9.

5. A method for assaying interferon-γ production from a sample comprising T cells from a subject, comprising
i. contacting the sample comprising T cells from the subject with five polypeptides comprising SEQ ID NOS: 1, 3, 5, 7 and 9; and
ii. detecting the presence of interferon-γ produced by the T cells.

## Patentansprüche

1. Verfahren zum Nachweis von Antikörpern in einer Probe von einem Subjekt, wobei die Antikörper an Epitope von *Mycobacterium tuberculosis-Antigenen* binden, umfassend
i. Inkontaktbringen der Probe mit fünf Polypeptiden umfassend SEQ ID NOS: 1, 3, 5, 7 und 9; und
ii. Nachweis der Bildung von Antikörper-Peptid-Komplexen, die diese Polypeptide umfassen; wobei die Bildung der Komplexe das Vorhandensein von Antikörpern gegen Epitope von *Mycobacterium tuberculosis*-Antigenen in der Probe anzeigt.

2. Verfahren nach Anspruch 1, wobei das Subjekt mit *Mycobacterium tuberculosis* infiziert ist.

3. Verfahren nach einem der Ansprüche 1 bis 2, wobei die Probe ausgewählt ist aus der Gruppe bestehend aus Blut, Serum, Plasma, Lymphknoten, Haut, Speichel, Urin, Zerebrospinalflüssigkeit und Milch.

4. Vorrichtung zum Testen auf das Vorhandensein von Antikörpern in einer Probe von einem Subjekt, wobei die Antikörper an Epitope *von Mycobacterium tuberculosis*-Antigenen binden, wobei die Vorrichtung fünf Polypeptide umfasst, die SEQ ID NOS:1, 3, 5, 7 und 9 umfassen.

5. Verfahren zum Testen der Interferon-γ-Produktion aus einer Probe, die T-Zellen von einem Subjekt umfasst, umfassend
i. Inkontaktbringen der Probe, die T-Zellen von dem Subjekt umfasst, mit fünf Polypeptiden umfassend SEQ ID NOS:1, 3, 5, 7 und 9; und
ii. Nachweis des Vorhandenseins von Interferon- γ, das von den T-Zellen produziert wird.

## Revendications

1. Procédé de détection d'anticorps dans un échantillon provenant d'un sujet, dans lequel les anticorps se lient à des épitopes d'antigènes de *Mycobacterium tuberculosis,* comprenant les étapes consistant à
i. mettre en contact l'échantillon avec cinq polypeptides comprenant les SEQ ID NO : 1, 3, 5, 7 et 9 ; et
ii. détecter la formation de complexes anticorps-peptide comprenant lesdits polypeptides ;
dans lequel la formation desdits complexes est indicative de la présence des anticorps à des épitopes d'antigènes de *Mycobacterium tuberculosis* dans ledit échantillon.

2. Procédé selon la revendication 1, dans lequel le sujet a été infecté par *Mycobacterium tuberculosis.*

3. Procédé selon l'une quelconque des revendications 1 à 2, dans lequel l'échantillon est choisi dans le groupe constitué de sang, sérum, plasma, ganglions lymphatiques, peau, salive, urine, liquide céphalo-rachidien et lait.

4. Dispositif pour tester la présence d'anticorps dans un échantillon provenant d'un sujet, dans lequel les anticorps se lient à des épitopes d'antigènes de *Mycobacterium tuberculosis,* dans lequel le dispositif comprend cinq polypeptides comprenant les SEQ ID NO : 1, 3, 5, 7 et 9.

5. Procédé pour tester la production d'interféron-y à partir d'un échantillon comprenant des lymphocytes T provenant d'un sujet, comprenant les étapes consistant à
i. mettre en contact l'échantillon comprenant des lymphocytes T du sujet avec cinq polypeptides comprenant les SEQ ID NO : 1, 3, 5, 7 et 9 ; et
ii. détecter la présence d'interféron-y produit par les lymphocytes T.
